# EUROPEAN PATENT SPECIFICATION

(11) **EP 3 837 544 B1**
(45) Date of publication and mention of the grant of the patent: **01.04.2026**
(21) Application number: 19849778.6
(22) Date of filing: 16.08.2019
(51) Int. Cl.: G01N 33/49, B01D 69/02, B01D 71/02

(54) **DEVICES AND METHODS FOR SAMPLE ANALYSIS USING MICROSLIT FILTERS**
VORRICHTUNGEN UND VERFAHREN ZUR PROBENANALYSE UNTER VERWENDUNG VON MIKROSPALTFILTERN
DISPOSITIFS ET PROCÉDÉS POUR L'ANALYSE D'ÉCHANTILLON AU MOYEN DE FILTRES À MICROFENTE

(30) Priority: 16.08.2018 US 201862719013 P; 02.11.2018 US 201862754946 P
(43) Date of publication of application: 23.06.2021
(73) Proprietor: SiMPore Inc., West Henrietta, NY 14586 (US)
(72) Inventor: CARTER, Jared A., Rochester, NY 14623 (US); ROUSSIE, James A., Rochester, NY 14617 (US)
(74) Representative: FRKelly
(86) International application number: PCT/US2019/046947
(87) International publication number: WO 2020/037288

(56) References cited:
- WO-A1-2016/175985
- WO-A1-2017/186129
- WO-A1-2019/036545
- US-A1- 2011 244 443
- US-A1- 2014 154 731
- US-A1- 2015 118 728
- US-A1- 2015 118 728
- US-A1- 2016 146 823
- US-A1- 2016 146 823
- US-A1- 2016 209 320
- VANDERMEERSCH GRIET ET AL: "A critical view on microplastic quantification in aquatic organisms", ENVIRONMENTAL RESEARCH, ACADEMIC PRESS, SAN DIEGO, CA, US, vol. 143, 3 August 2015 (2015-08-03), pages 46 - 55, XP029307410, ISSN: 0013-9351, DOI: 10.1016/J.ENVRES.2015.07.016

## Description

### Field of the Disclosure

This disclosure relates to the enumeration of microplastic components within a sample of interest, and more particularly, the capture of such components by efficient isolation using microslit filters with high permeation capacity and precision molecular cut-off characteristics.

### Background of the Disclosure

The enumeration of a desired species of interest, such as, for example, cells or other components, within a liquid sample is a critical step required for research and clinical analytical purposes. Enumeration may comprise the counting of, or determining concentration of, such components in a liquid sample.

While many methods have been developed for these purposes, the most widely used method involves passing the species of interest through a single aperture for its enumeration, wherein electrical signals are used to count species passage events (in order to determine concentration) and to size the species as well.

One well-known problem associated with single aperture component counters is their tendency to clog (e.g., become irreversibly filled with species so that they no longer pass any additional species). This clogging phenomenon is often due to the complexity of the fluid matrix and the highly abundant concentration of species in the fluid samples often counted by the single aperture method. For example, complete blood cell counts (CBC) are performed by single aperture counters. However, the high abundance of hemocytes, as well as matrix factors (e.g., serum albumin), can clog the single aperture, distort its readings, and lead to down-time on the counter instrument. Similar problems are encountered when assessing the concentration of components in samples such as, for example, chemical products or industrial effluents or discharges, where a fluid sample is tested for concentration and size of particulate matter.

The problem associated with single aperture-based approaches is their limited capture area for species of interest. For purposes of this disclosure, it is understood that capture area relates to the amount of surface area or volume in which the species of interest are captured. Since the single aperture has a limited volume in which species of interest are captured for enumeration, its clogging problems can be associated with this aspect.

US2015118728A1 discloses an apparatus for separating a biological entity from a sample volume is provided. The apparatus includes an input chamber including an inlet configured to receive the volume sample, and an outlet, at least one magnetic element adjacent a portion of the input chamber, the magnetic element configured to provide a magnetic field in a vicinity of the portion of the input chamber to trap at least some leukocytes from the sample volume, and a filter in fluid communication with the outlet, the filter configured to separate the biological entity. According to further embodiments of the present invention, a method for separating a biological entity from a sample volume is also provided.

US2011244443A1 discloses the separation/capture of specific cells and/or contaminants, as well as the determination, monitoring, and treatment of cancer. Moreover, some embodiments are directed to methods, systems and devices for removing cancer, stem and/or tumor cells in vivo or in vitro from a bodily fluid to prevent or impede the proliferation of a cancer. Some embodiments provide a blood-compatible filter comprising, for example, a membrane provided with a number of openings (preferably precise) which yield minimal detrimental effect both quantitatively and qualitatively on cells present in the bodily fluid during the separation process.

US2016146823A1 discloses methods and apparatuses for analyzing particles in a sample. In some aspects, the particles can be analytes, cells, nucleic acids, or proteins and contacted with a tag, partitioned into aliquots, detected by a ranking device, and isolated. The methods and apparatuses provided herein may include a microfluidic chip. In some aspects, the methods and apparatuses may be used to quantify rare particles in a sample, such as cancer cells and other rare cells for disease diagnosis, prognosis, or treatment.

VANDERMEERSCH GRIET ET AL discloses "A critical view on microplastic quantification in aquatic organisms" in ENVIRONMENTAL RESEARCH, ACADEMIC PRESS, SAN DIEGO, CA, US, vol. 143, 3 August 2015, pages 46-55, XP029307410, ISSN: 0013-9351, DOI: 10.1016/J.ENVRES.2015.07.016.

What is needed is a method for the enumeration of components that is not prone to clogging as are single aperture-based methods.

### Brief Description of the Figures

For a fuller understanding of the nature and objects of the disclosure, reference should be made to the following detailed description taken in conjunction with the accompanying figures.
Figure 1 provides images of a representative microslit filter (B) and a representative fluidic device (A) incorporating such a microslit filter.
Figure 2 provides exemplary uses (A, B, C) of microslit filters for retaining and measuring biological (not claimed) and non-biological components from liquid samples.
Figure 3 provides exemplary uses (A, B, C, D) of microslit filters for retaining and measuring biological (not claimed) and non-biological components from liquid samples.
Figure 4 provides an example of a microslit filter with an underlying fluidic cavity (A), an example of a scheme for fluidically connecting multiple filtration membranes and intervening fluidic cavities (B), and an example of a fluidic rendering of a device for fluidically addressing a membrane system.
Figure 5 show panels (A, B, C, D, E, F) of images describing the design and modeling of an exemplary tangential flow fluidic device for incorporating microslit filters.
Figure 6 demonstrates the results (A, B, C) of a pressure drop analysis which evaluated microslit height.
Figure 7 shows resulting phase contrast microscopy images (A, B, C, D).
Figure 8 shows the results (A, C, D) from the selective retention by a microslit filter of a polymeric particle, and exemplary devices (B) of the present disclosure.
Figure 9 shows particle retention and permeation results (A) for three different nanoporous filters and a representative 100 nm-thick nanoporous filter (B) of the present disclosure.
Figure 10 shows particle retention and permeation results for two different microslit filters.
Figure 11 provides micrographic images (A, B) of microslit filters of the present disclosure.
Figure 12 shows particle retention and permeation results for a microslit filter of the present disclosure.
Figure 13 shows (A) transmission of various nanoparticle sizes through a microslit filter and a representative comparative polymeric filter, and a graph (B) showing the difference in fouling between a microslit filter and a representative comparative polymeric filter.
Figure 14 shows (A, B) a microslit filter of the present disclosure.
Figure 15 shows a phase contrast light microscopy image (A) of a microslit filter, and the same microslit filter showing an IR signature of microplastic particles retained on the membrane surface (B).
Figure 16 provides an exemplary ex situ analytical assay of the present disclosure, wherein infrared spectroscopy was used to identify the composition of a first type of particle (e.g., poly (methyl methacrylate) - PMMA) retained by a microslit filter.
Figure 17 provides an exemplary ex situ analytical assay (A, B, C) of the present disclosure, wherein infrared spectroscopy was used to identify the composition of a second type of particle (e.g., polyethylene) retained by a microslit filter.
Figure 18 provides (A, B, C) an exemplary use of a transmembrane pressure-dependent method of the present disclosure for a first type of biological component (not claimed) (e.g., Adenovirus) and compares the method between a microslit filter and a representative polymeric filter.
Figure 19 provides (A, B) an exemplary use of a transmembrane pressure-dependent method of the present disclosure for a second type of biological component (not claimed) (e.g., Maraba virus) and compares the method between a microslit filter and a representative polymeric filter.
Figure 20 provides an exemplary use of a transmembrane pressure-dependent method of the present disclosure for a third type of biological component (not claimed) (e.g., B. diminuta bacteria) and compares the method between a microslit filter and a representative polymeric filter.
Figure 21 provides micrographic images and physical dimensions of representative nanoporous and microslit filters of the present disclosure.
Figure 22 provides various membrane physical properties of nanoporous and microslit filters of the present disclosure.
Figure 23 provides micrographic images (A, B, C, D) of microslit and microporous filters of the present disclosure.
Figure 24 provides micrographic images (A, B, C) of microporous filters of the present disclosure.
Figure 25 provides micrographic images (A, B, C, D, E, F) of nanoporous filters of the present disclosure.
Figure 26 provides micrographic images of microslit filters of the present disclosure.

### Detailed Description of the Disclosure

Ranges of values are disclosed herein. The ranges set out a lower limit value and an upper limit value. Unless otherwise stated, the ranges include all values to the magnitude of the smallest value (either lower limit value or upper limit value) and ranges between the values of the stated range.

Provided is a method for enumerating and determining the composition of microplastic components of interest from a liquid sample, the method comprising :
a) contacting the liquid sample with a microslit filter, such that the one or more microplastic components of interest are retained by the microslit filter and undesired components permeate through the microslit filter;
b) optionally, washing the retained microplastic components of interest; and
c) measuring the quantity of retained microplastic components of interest to determine the concentration of the retained components of interest in the liquid sample.

The claimed method further comprises performing one or more first analytical assay on the one or more retained microplastic components of interest disposed on the microslit filter, wherein the components of interest are retained by the microslit filter during the first analytical assay, wherein the first analytical assay comprises determining the composition of the retained components of interest using infrared spectroscopy and/or Raman spectroscopy; wherein the microslit filter comprises a silicon nitride membrane and wherein in the case of infrared spectroscopy the microslit filter comprises an infrared reflective layer comprising metal selected from Au, Pt, Ag, Ir, Rh and combinations thereof or in the case of Raman spectroscopy the microslit filter comprises a Raman-silent layer comprising MgF₂ or CaF₂.

The liquid sample bearing components of interest may be a biofluid, which may include, but are not limited to, cell lysates, venous or arterial whole blood, plasma, serum, sputum, urine, cerebrospinal fluid, conditioned cell culture media or other fluids derived from cell culture and other fluids containing molecules of biological origin, or any solutions in contact with biological tissues (such as, for example, bodily secretions, discharges, and/or excretions, as well as swabs and/or aspirates of bodily tissues, among others, ), or any combination thereof. The components of interest are microplastics. Examples of components of interest may further include, but are not limited to, acrylonitrile butadiene styrene, polyester, polyethylene, polymethylmethacrylate, polyethylene terephthalate, polyvinyl chloride, polypropylene, polylactic acid, polycarbonate, polyoxymethylene, aliphatic- and aromatic-substituted polyamides or polyimines, polytetrafluoroethylene, or per- and poly-fluoroalkyl polymers, polyketones, polyglycols, polypeptides, among other polymers. Other examples of components of interest may further include, but are not limited to, silicon-based or carbon-based fibers. Of course, other possible types of non-biological components are possible and these examples have been provided merely for exemplary purposes.

Non-biological liquid samples that are compatible with the present disclosure include, but are not limited to, samples of water, industrial discharges, food products, milk, air filtrates, among others, and thus include, but are not limited to, food, environmental and industrial samples.

For purposes of this disclosure, it is understood that a plurality of desired components is retained by the microslit filter, while a plurality of undesired components permeates the microslit filter, upon the filtration step (e.g., of a)).

In various examples, the liquid sample can bear populations of both biological components and non-biological components. The methods disclosed herein (e.g., steps a) through c)), in combination with any optional pre-treatment step, can be used to measure the concentration of the one or more populations of biological and non-biological components, and further, the one or more analytical assays described hereunder can be optionally used to determine the composition of such populations of both components. The claimed method determines the composition of microplastics.

The filtration (e.g., of a) can be performed by contacting the liquid sample with a microslit filter of specified characteristics. For example, the width of the microslit filter's openings can be specified to retain desired components of a certain diameter and permeate other undesired components of another diameter. As one example, the microslit filter can have either cubic prism or rectangular prism openings, where the width of such prism-like openings is specified relative to the diameter of spherical components of interest in the liquid sample. Accordingly, the width of the microslit filter's openings must be specified in order to affect the desired retention or permeation outcomes.

To retain a desired component of a certain diameter, the retained components' diameter must be greater than the width of the microslit filter's openings. Accordingly, it is recognized that retention of such components is desired as an isolation for their subsequent enumeration by the methods disclosed herein. By contrast, to permeate an undesired component of another diameter (e.g., smaller diameter), the permeated components' diameter must be less than the width of the microslit filter's openings. As one example, a microslit filter can be specified to capture polymeric components of a certain diameter (and larger) out of the effluent of a waste water treatment plant and to permeate other smaller species in the effluent, such that the retained polymeric components are likely to comprise a range of component diameter that are equal and greater than the opening width of the microslit filter used for the filtration. Capture and retention of polymeric components is desired so that the number of such species can be determined from a known volume of the liquid sample. Of course, other component types can be captured and retained by the methods disclosed herein and these examples have been provided merely for exemplary purposes.

The filtration (e.g., of a)) can be performed by several flow modalities. For example, the filtration comprises dead-end or normal flow initiated by gravity, hydrostatic pressure, pumping, vacuum, centrifugation, or gas pressurization, wherein the liquid sample is introduced to the cis-side of the microslit filter and translocates to the opposing trans-side of the microslit filter. In another example, the filtration comprises tangential flow initiated by liquid sample alone or in combination with a buffer solution, respectively, on opposing cis and trans-sides of the microslit filter. The tangential flow modality may further comprise diafiltration, wherein a specified transmembrane pressure vector drives both capture/retention of desired components of interest and permeation of undesired components through the microslit filter, while a specified tangential flow vector parallel to the microslit filter surface drives downstream flow of non-captured/retained components within the openings of the microslit filter (thereby reducing clogging or fouling). It should be recognized that, in examples wherein tangential flow is used, some proportion the larger, desired components will be swept downstream by the tangential flow vector parallel to the microslit filter surface and that some proportion of the smaller, undesired components will be similarly swept downstream (and not permeate through the microslit filter).

In some examples, one or more washing steps can follow a) in order to remove nonspecifically and undesired components associated with the retained components of interest. The wash solution may be any buffer or solution appropriate to the nature and composition of the components of interest and to that of the matrix components of the liquid sample. Other buffers, solutions, and the like may be more appropriate for non-biological components (e.g., polar or non-polar solvents). Of course, other washing conditions are possible and these examples have been provided merely for exemplary purposes. Similar flow modalities as described for the filtration of a) may be used for any washing steps. Any such washing steps, if included, may remove smaller, non-desired components and species (e.g., matrix components and the like) from any retained components and such non-desired components or species may be transferred to a waste chamber or vessel as part of any washing steps. Moreover, if such washing steps are used in a tangential flow configuration, they may promote removal of non-specifically bound undesired components by both permeation and downstream flow.

In some examples, an optional upstream sample preparation step may be additionally used. In addition to the examples previously described, a liquid sample may be contacted by a first microslit filter, wherein the openings in this first microslit filter are specified to reject undesired components (e.g., components with a diameter larger than the rectangular prism openings of a microslit filter), and to permeate the desired components of interest, which are then subsequently contacted by a second microslit filter (e.g., the microslit filter of a)). In these examples, the outflow of the first microslit filter is in fluid contact with the second microslit filter, such that the permeate of the first filter can be contacted by the second microslit filter.

In the various examples of the methods disclosed herein, the microslit filter-based format may overcome well-known problems of single aperture-based formats for determining the number of components of interest in a liquid sample. For example, a microslit filter offers greater surface area and volume over that of a single aperture, since the microslit filter provides a plurality of openings (e.g., apertures) that do not foul and clog like the limited surface area and volume of a single aperture. If a microslit filter becomes occluded anywhere along the length of its openings, there remains additional surface area and volume along the remaining length of its openings for continued operation. By contrast, once a single aperture becomes clogged with a species, it can no longer permeate additional species and continue its usual mode of operation. In addition, the straight-through, cubic or rectangular prism openings of microslit filters (rather than a cylindrical opening of a single aperture) enables low permeation resistance filtration properties. The low permeation resistance of microslit filters is a consequence of their thickness, aspect ratio of prism-like openings, and minimal internal surface area. This combination of properties endows microslit filters with high filtration capacity; e.g., the ability to permeate a large number of undesired components without clogging. Furthermore, the specified aspect ratio of the prism openings of microslit filters enables selective filtration outcomes; e.g., the ability to retain desired components of interest in a selective manner, while permeating undesired components. Finally, it is recognized that the permeation resistance of microslit filters is proportional to its thickness (e.g., lower membrane thickness provides low permeation resistance), but is inversely proportional to its porosity (e.g., higher porosity provides low permeation resistance). Accordingly, the methods of the present disclosure are further enabled by the combination of low thickness (e.g., ≤ 1 µm membrane thickness) and high porosity (e.g., ≥ 10%) of the exemplary microslit filters disclosed herein. For purposes of this disclosure, porosity refers to the plurality of openings (e.g., apertures) through the membrane per unit of membrane surface area.

The filtration (e.g., of a)) can be performed at a range of pressurization that is compatible with maintaining the integrity of species in a liquid sample. This compatibility relies on the low permeation resistance property of microslit filters. For example, the pressurization can be from 10 Pa to 1.0 kPa and all Pa values therebetween. In another example, the pressurization can be that only applied by hydrostatic pressure. In some examples, wherein the sample is a biofluid and the components of interest are cells (e.g., hemocytes of whole blood and the like), the pressurization can be sufficiently low that the shear forces applied to cells when permeating the microslit filters do not cause cell lysis. For example, a microslit filter of 8 x 50 µm openings and 400 nm thickness (membrane thickness) applies a maximum of 18 dynes/cm² at 1.0 kPa, while 1,500 dynes/cm² is known, for example, to cause erythrocyte lysis (Leverett et al, 1972).

To determine the number of captured and retained components on the microslit filter, a known volume of liquid sample is passed via the flow modalities described herein, the number of retained components assessed by the measuring methods described herein, and the concentration of components calculated by dividing the number of retained components by the known volume of liquid sample that was passed. In some examples, a fluidic device is configured to pass a known volume of liquid sample. In another example, a flow meter within a fluidic device measures the volume of passed liquid sample. In various examples, an algorithm is used to record retained components, to tracked volume of liquid sample passed and to calculate component concentration. In some examples, such calculations may be performed on repeat or replicate measurements of the same component of interest; for instance, the component can be captured and retained on multiple microslit filters within one fluidic device, and an algorithm is used to calculate the arithmetic mean of the multiple instances of retained components. In such examples, the reported component concentration is the average of the data collected from the multiple instances. Alternatively, a single instance of measured component retention and volume passage is used to calculate the component concentration, which can be reported as a single datum.

The step of determining the number of retained components (e.g., step c)) by any of the measurement methods disclosed herein can be performed as either in situ or ex situ measurements, with respect to the fluidic devices used for the contacting and optional washing steps (e.g., steps a) and b), respectively). As an example of an in situ measurement, the microslit filter remains within a fully assembled or partially disassembled fluidic device when performing the measurement. In this example, the fluidic device may be transferred into an instrument for carrying out any measurement. In an example of an ex situ measurement, the microslit filter is extracted from a partially or fully disassembled fluidic device and the microslit filter is transferred to an instrument for carrying out any measurement. In this latter example, the extracted microslit filter may be optionally transferred to another substrate prior to transfer into the instrument carrying out the measurement.

The measuring method performed on the retained components (e.g., of c)), to determine the number of retained components, can be carried out by a variety of modalities and methods. For example, the measuring methods can include, but are not limited to, optical imaging, electronic interrogation, and optical diffraction modalities. For purposes of this disclosure, both optical imaging and optical diffraction, as well as other optically dependent first and second analytical assays disclosed hereunder, are considered collectively as optical modalities or as optical methods. Such measurements can be performed in a single instance or as replicate instances and their results reported as a single datum or arithmetic mean values.

The measuring method performed on the retained components (e.g., of c)), to determine the number of retained components, can also be carried out by a transmembrane pressure-dependent modality and method. For example, the measuring methods can include a change or differential measurement for the transmembrane pressure across a microslit filter or microslit membrane. Such measurements can be performed in a single instance or as replicate instances and their results reported as a single datum or arithmetic mean values.

In various examples, the measuring method performed on the retained components (e.g., of c)) to determine their concentration can be a combination of optical imaging, electronic interrogation, optical diffraction, and transmembrane pressure modalities. for example, the measuring method can be a combination of optical imaging and transmembrane pressure methods. In another example, the measurement can be a combination of optical imaging and electronic interrogation. In various examples, the two or more measurement methods can be performed one after the other (in any temporal order) or may be performed simultaneously. The results from such combined measurements may be reported as single instances, arithmetic means, and/or as correlated data between the two or more measurement methods.

In an example, the step of determining the number of retained components can comprise optical imaging. For example, captured and retained components may be counted using an illuminating light source and a light detector and the number of components counted by an algorithm that processes the resultant images (e.g., a signal processing algorithm). In some examples, a physical property of a particular component type may be used to determine the number of retained components (e.g., fluorescence, luminescence, absorbance, and the like, and combinations thereof). For example, the hemoglobin content within retained erythrocytes may be used as the basis of an absorbance optical signal at a specified wavelength for determining the number of retained erythrocytes. As another example, a fluorescent signal of a cadmium selenide quantum dot, generated at a specified excitation wavelength and recorded at a specified emission wavelength, may be used. A light source and a detector may be used for optical imaging, excitation and recording of emission, luminescent and/or absorbance signals, and may further include a light source and a detector, in a variety of fashions including photodiode arrays, charge coupled devices, and other optical sensing techniques for carrying out the optical signal detection methods. Of course, other physical properties, as well as detection modalities, may be used and these examples have been provided merely for exemplary purposes.

In other examples, the optical imaging can further comprise one or more plasmonic-enhanced optical imaging modality; e.g., surface plasmon resonance, plasmon- or surface-enhanced fluorescence, surface-enhanced Raman spectroscopy, and the like. As known to those skilled in the related art, incident light may plasmonically excite molecules on retained components, and thus upon optical interrogation, amplify emission spectra and improve limit of detection and sensitivity. Further, surface plasmon resonance may rely on a shift in refractive index caused by such plasmon excitation, but may be affected by ambient temperature. In some examples of plasmonic detection, microslit filters may be conformally coated with a noble metal that is plasmonically active (e.g., Au, Pt, Ag, Ir, Rh, and the like, and combinations thereof).

In some examples comprising an optical imaging modality, microslit filters may be conformally coated to reduce any signal contributed by the microslit filter with respect to the optical imaging modality; that is, the coating may reduce background signal and thus improve signal-to-noise (or resolution and sensitivity) of the optical imaging. In some examples, the coating may be a conformal coating and may include, but is not limited to, Ti, Cr, Al, Au, Pt, Ag, Ir, Rh (and the like, as well as various combinations thereof), or MgF₂, CaF₂, TiO₂ (and the like, as well as various combinations thereof). In one example, the optical modality is Fourier-transformed infrared spectroscopy and the microslit filter is coated with a metal (e.g., Al, Au, or Ag) and such metal coating comprises an infrared light reflective layer. In another example, the optical modality is Raman spectroscopy and the coating is a Raman-silent layer (e.g., MgF₂, CaF₂, or TiO₂). The thickness of the coating may be specified to affect the desired property (e.g., infrared reflectivity or Raman silence). Accordingly, the coating may have a range of thickness; for example, the thickness can be from 10 nm to 1,000 nm, including all values to the nm and ranges therebetween. Of course, other combinations of microslit filter coatings and optical modalities are possible and these examples have been provided merely for exemplary purposes.

In various examples, the measurement (e.g., of c)) can further comprise an electronic interrogation modality based on transmembrane electrical resistance or transmembrane impedance, among other possibilities. For example, upon retention of desired components by a microslit filter, the openings of such filters are occluded, such that the transmembrane electrical resistance to an input current increases or is blocked altogether. One or more pairs of electrodes may be used to measure the transmembrane electrical resistance, if placed on opposing cis- and trans-sides of a microslit filter. As another example, a function generator may be used to generate an input current at high frequency, such that transmembrane impedance may be recorded. The impedance of an interface is generally determined by applying a sinusoidal voltage perturbation, while simultaneously recording the current response using a voltmeter. A linear voltage-current response may be obtained by small (e.g., ~10 mV peak to peak) perturbations. Such voltage-current responses thus provide the related transmembrane impedance. One or more pairs of electrodes may be used to measure such transmembrane impedance, in connection with a function generator and voltmeter, if the electrodes placed on opposing cis- and trans-sides of a microslit filter. In such examples of electronic interrogations, it is understood that the extent of microslit filter occlusion by retained components of interest is directly correlated with any changes in transmembrane electrical resistance or impedance, thus such electronic interrogations can be used to determine the number of components that were retained. Of course, other electronic interrogation methods are possible and these examples have been provided merely for exemplary purposes.

In the various examples of the electronic interrogation methods disclosed herein, the microslit filter-based format may overcome well-known sensitivities of such methods to the presence of electrolytes. For example, most biofluid samples comprise 1 to 200 mM salt concentration. Such salt concentration may deleteriously affect the detection limits of electronic interrogation methods. However, the salt, pH, and/or other electrolyte concentrations may be specified by the one or more filtration and optional washing steps of the methods disclosed herein. For example, salt concentration may be easily altered subsequent to capture and retention of components of interest, by contacting with buffer solutions of specified salt that improves such electronic interrogations.

In various examples, the measurement (e.g., of c)) can further comprise an pressure-dependent modality based on pressure across the microslit filter (e.g., transmembrane pressure). For example, upon retention of desired components by a microslit filter, the openings of such filters are occluded, either partially or fully, such that the fluidic resistance (e.g., resistance against further flow) increases and thus can be measured as an increase in transmembrane pressure. One or more pressure sensors may be used to measure the transmembrane pressure, if placed on opposing cis- and trans-sides of a microslit filter. In such examples, it is understood that the extent of microslit filter occlusion by retained components of interest is directly correlated with any changes in transmembrane pressure, thus such pressure-dependent modalities can be used to determine the number of components that were retained. Of course, other pressure-dependent modalities or methods are possible and these examples have been provided merely for exemplary purposes.

In various examples of the optical imaging and electronic interrogation methods, a signal processing algorithm can be used to record, collect, and compare the optical and electronic data for purposes of detecting the retention of components of interest. Further, the extent of the differences between sample (i.e., used in filtration) and reference (i.e., not used in filtration) microslit filters may be used to quantitate the retention of components of interest within a liquid sample and may require the generation of a series of reference data. Each instance of the series would be recorded, wherein a known number of model components retained by microslit filters are used in each instance of the series. The sample microslit filter data would be compared to the reference series in order to determine the number of components within the liquid sample. A linear regression or other appropriate statistical method may be employed for the comparison of the sample to the reference data. As one example, the signal processing algorithm may compare the intensity or magnitude of fluorescent, absorbance, or electronic data, wherein any differences in the intensity of any such data between reference and sample comprises a potential measure for quantitating the number of components of interest that may be retained by a microslit filter. In this example, it is assumed that retained components that change any such fluorescent, absorbance, or electronic data are dependent on microslit filter occlusion. Thus in some examples, one or more additional reference microslit filters may be used for optical imaging and electronic interrogation methods.

In various examples of the pressure-dependent methods, a signal processing algorithm can be used to record, collect, and compare the pressure data for purposes of detecting the retention of components of interest. Further, the extent of the differences between sample (i.e., used in filtration) and reference (i.e., not used in filtration) microslit filters may be used to quantitate the retention of components of interest within a liquid sample and may require the generation of a series of reference data. Each instance of the series would be recorded, wherein a known number of model components retained by microslit filters are used in each instance of the series. The sample microslit filter data would be compared to the reference series in order to determine the number of components within the liquid sample. A linear regression or other appropriate statistical method may be employed for the comparison of the sample to the reference data. As one example, the signal processing algorithm may compare transmembrane pressure values, wherein any differences in the magnitude of such values between reference and sample comprises a measure for quantitating the number of components

In a further example, the signal processing algorithm may permit the real-time detection of components of interest. For example, quantitative changes in fluorescence, absorbance, or electronic data (when comparing reference and sample microslit filters) may be used in real-time measurements as components are retained by a microslit filter during the filtration step. In this example, any of the flow modalities disclosed herein would be used to capture any components in real-time, and the optical or electronic interrogation method of the present disclosure used to quantitatively detect components as they are retained.

In a further example, the signal processing algorithm may permit the real-time detection of components of interest by a pressure-dependent modality. For example, quantitative changes in transmembrane pressure (when comparing reference and sample microslit filters) may be used in real-time measurements as components are retained by a microslit filter during the filtration step. In this example, any of the flow modalities disclosed herein would be used to capture any components in real-time, and the pressure-dependent methods of the present disclosure used to quantitatively detect components as they are retained.

In other examples, the step of determining the number of retained components can further comprise an optical diffraction modality, wherein such modalities may include, but are not limited to, recording the optical diffraction spectra of microslit filters after the filtration step to observe any captured and retained components of interest.

In an example, a further method for enumerating components of interest from a liquid sample further comprises contacting the liquid sample with a sample microslit filter, such that the components of interest are retained by the microslit filter and undesired components permeate through the microslit filter, optionally, washing the retained components, recording the optical diffraction spectrum of the sample microslit filter (e.g., a sample optical diffraction spectrum), and comparing the sample optical diffraction spectrum to an optical diffraction spectrum of a reference optical diffraction spectrum (e.g., the native microslit filter).

In examples of the optical diffraction method, detecting the retained components is dependent on observable differences between the sample and reference optical diffraction spectra. In its native state (e.g., not used in filtration), the periodic openings of microslit filters can cause coherent light to be diffracted and a repeatedly observable diffraction pattern or spectrum generated. These consistent diffraction spectra can be generated upon trans-illumination with a coherent light source (e.g., laser of specified wavelength) and thus are well-suited to serve as a reference for comparative purposes. However, the retention of components by the sample microslit filter disrupts its periodicity and thus distorts its optical diffraction upon trans-illumination with the same coherent light source. Therefore, observable differences between the sample and reference spectra can be indicative of the presence of the retained components on the sample microslit filter.

In some examples of the optical diffraction method, a signal processing algorithm can be used to record, collect, and compare the sample and reference optical diffraction spectra for purposes of detecting the retention of components of interest. The extent of the differences between sample and reference optical diffraction spectra may be used to quantitate the retention of components of interest within a liquid sample and may require the generation of a series of reference optical diffraction spectra. Optical diffraction spectra from each instance of the series would be recorded, wherein a known number of model components retained by microslit filters used in each instance of the series. The sample optical diffraction spectrum would be compared to the reference series in order to determine the number of components within the liquid sample. A linear regression or other appropriate statistical method may be employed for the comparison of the sample spectrum to the reference series spectra. As one example, the signal processing algorithm may compare the intensity or magnitude of first, second, third, and successive diffraction order peaks, wherein any differences in the intensity of any such diffraction order peaks between reference and sample diffraction spectra comprises a potential measure for quantitating the number of components of interest that may be retained by a microslit filter. In this example, it is assumed that retained components that disrupt the periodicity of trans-illuminated microslit filters may cause quantitative changes in diffraction order peak intensities (e.g., reduction in some peaks, appearance of new diffraction order peaks, or increases in other diffraction order peaks), and that these diffraction order peak changes may be observed when comparing reference and sample diffraction spectra.

In a further example, the signal processing algorithm may permit the real-time detection of components of interest. For example, quantitative changes in diffraction order peaks (when comparing reference and sample diffraction spectra) may be used in real-time measurements as components are retained by a microslit filter during filtration. In this example, any of the flow modalities disclosed herein would be used to capture any components in real-time, and the optical diffraction method of the present disclosure used to quantitatively detect components as they are retained.

In an aspect, a further method for enumerating components of interest from a liquid sample further comprises sorting two or more desired components of interest by selective retention of such two or more populations of components and using one of the measuring methods disclosed herein to quantitate the number of such components. This further method comprises sorting the two or more components of interest into distinct populations based on a physical property of such components (e.g., density, size, or diameter). The sorting of two or more sets of components into distinct populations makes use of two or more specified microslit filters or microslit filter elements.

In a further example, a further method for enumerating components of interest from a liquid sample further comprises:
d) contacting the liquid sample with two or more microslit filters, such that the first population of desired components of interest is retained by a first microslit filter, a second population of desired components of interest is retained by a second microslit filter, and any successive populations of components are retained by successive microslit filters, and any undesired component populations permeate through the microslit filters;
e) optionally, washing the retained components; and
f) measuring the quantity of the two or more populations of retained components to determine their concentration in the liquid sample.

The filtration (e.g., of d)), wherein the two or more components of interest are sorted into distinct populations, may be performed based on the physical properties of each distinct component population (e.g., density, size or diameter, among others) with respect to the filtration properties of the two or more microslit filters. For example, a first microslit filter having rectangular prism openings corresponding in width to the diameter of a first set of components may be used for selective capture and retention of this first component population. A second microslit filter having rectangular prism openings corresponding in width to the diameter of a second set of components may be used for selective capture and retention of this second component population. Any third and successive component populations may be sorted by additional microslit filters with openings corresponding to the diameter of any such third and successive component populations. Of course, microslit filter opening width and component diameter are two of several possibilities and these examples have been provided merely for exemplary purposes.

**In** examples of the further method, the successive microslit filters may be configured in series (e.g., of different fluidic planes) or in parallel (e.g., of the same fluidic plane), such that the outflows from a preceding microslit filter are in fluidic contact with the inflows of subsequent microslit filters.

For successive microslit filters configured in parallel, the downstream flow of the sample along the parallel fluidic plane allows a first component population to be retained at a first microslit filter, a second component population to be retained at a second microslit filter, and any successive component populations by successive microslit filters, such that the downstream flow of the sample carries components from one microslit filter to successively configured microslit filters.

**In** various examples of the further method, the liquid sample can bear populations of both biological components and non-biological components. The further methods disclosed herein (e.g., steps d) through f)), in combination with any optional pre-treatment step, can be used to measure the concentration of the one or more populations of biological and non-biological components, and further, the one or more analytical assays described hereunder can be optionally used to determine the composition of such populations of both components.

**In** examples of the further method, the filtration (e.g., of d)) can be performed using any of the flow modalities previously described for the filtration (e.g., of a)). Any optional washing step (e.g., of e)) can be performed using any of the flow modalities previously described for the optional washing step (e.g., of b)). Further, any of the measuring methods (e.g., of f)) can be performed using any of the modalities previously described for such steps (e.g., of c)). Such measurements can be performed as a single instance or as replicate instances, and reported as a single datum or as mean data.

In an aspect, a further method for enumerating components of interest from a liquid sample further comprises contacting a liquid sample with a microslit filter, wherein the microslit filter comprises a fluidic cavity proximally underlying its filter element, permeating the desired components of interest into this fluidic cavity where they are captured, optionally washing the microslit filter, and measuring the number of captured components within the fluidic cavity by the one or more methods disclosed herein. The openings in the microslit filter are specified for selective permeation of the desired components of interest into the fluidic cavity. Such microslit filter and underlying fluidic cavity structures are disclosed in Striemer et al. (U.S. Pat. Appl. No. 62/248,467). It should be noted that for examples related to this aspect of the disclosure, the permeating components are the desired components of interest (unlike other examples previously disclosed herein, where the permeating components were generally undesired components).

In an example, a further method for enumerating components of interest from a liquid sample further comprises:
g) contacting the liquid sample with a microslit filter, such that the components of interest permeate through the openings of the microslit filter and are captured within a fluidic cavity, while undesired components are not captured within said fluidic cavity;
h) optionally, washing the retained components; and
i) measuring the quantity of retained components to determine their concentration in the liquid sample.

The filtration (e.g., of g)), wherein the components of interest selectively permeate through the microslit filter and are captured within fluidic cavities proximally underlying the microslit filter, may be performed based on the physical properties of a component (e.g., density, size or diameter, among others) with respect to the filtration properties of the microslit filters. For example, a microslit filter having rectangular prism openings may be specified such that the width of its openings is larger than the diameter of a component of interest, thus permitting the component to permeate the microslit filter's openings and be captured in its underlying fluidic cavity. Of course, microslit filter opening width and component diameter are two of several possibilities and these examples have been provided merely for exemplary purposes.

In various examples of the further method, the liquid sample can bear populations of both biological components and non-biological components. The further methods disclosed herein (e.g., steps g) through i)), in combination with any optional pre-treatment step, can be used to measure the concentration of the one or more populations of biological and non-biological components, and further, the one or more analytical assays described hereunder can be optionally used to determine the composition of such populations of both components.

The filtration (e.g., of g)), wherein the components of interest selectively permeate through the microslit filter and are captured within fluidic cavities proximally underlying the microslit filter, must consider the range of component sizes within a liquid sample in order to affect the desired filtration outcome. For example, a microslit filter having rectangular prism openings may be specified such that the width of its openings permeates the desired components of interest, but also undesired components that are smaller (e.g., smaller diameter) than that of the desired components of interest. Therefore, the subsequent capture of such a mixture of components would complicate the measurement to determine the number of a particular component of interest (e.g., a mixture problem). Accordingly, as one example to mitigate against this mixture problem), the previously disclosed methods for sorting components into distinct populations (e.g., steps d)-f)) may be used in combination with microslit filters comprising a structure with two microslit filter elements (or two filtration membranes) separated by an intervening fluidic cavity. Such structures with two filtration membranes in one monolithic structure are disclosed in Striemer et al. (U.S. Pat. Appln. No. 62/248,467). In one example, a liquid sample is contacted by a microslit filter with a first filtration membrane, the components of interest permeate into a fluidic cavity proximally underlying this first filtration membrane, where such components are captured and retained by a second filtration membrane (e.g., a first distinct component population), and the one or more measurement methods of the present disclosure used to determine the number of components captured in the fluidic cavity that is disposed between the two filtration membranes (e.g., the intervening fluidic cavity). In this example, the rectangular prism openings in the first filtration membrane are specified to permeate the desired components of interest based on a physical property (e.g., diameter), along with other undesired components (e.g., smaller diameter components). The openings in the first filtration membrane are also specified to reject components larger than the size of the first instance of the components of interest. The rectangular prism openings in the second filtration membrane are specified to permeate undesired components based on a physical property (e.g., smaller diameter than the desired components). Successive microslit filters may be used to capture second, third, and any successive distinct component populations by configuring two or more filtration membrane-microslit filter structures in series, such that the outflow of the second filtration membrane of the first microslit filter is in fluidic contact with the first filtration membrane of a second microslit filter, and any successive first membranes of successive microslit filters are in fluid contact with second filtration membranes of subsequently configured microslit filters.

In the various examples, the successively configured microslit filters may be in fluidic connection by a variety of combinations. For example, all of the cis-sides of two or more microslit filters may be in fluid connection, such that the outflow (e.g., sample and those components not retained by the first microslit filter) flow downstream to the inlet of two or more microslit filters. In such an example, the two or more microslit filters would be configured in parallel. In another example, the trans-side of a first microslit filter is in fluidic connection with the cis-side of a microslit filter, such that the outflow of the first microslit filter (e.g., permeate of fractionated sample and any of its components not retained by the first microslit filter) flows to the inlet of the second microslit filter. In this latter example, the two or more microslit filters would be configured in series. Of course, many other configurations are possible and these examples have been merely provided for exemplary purposes.

In examples of the further method, the filtration (e.g., of g)) can be performed using any of the flow modalities previously described for the filtration (e.g., of a)). Any optional washing step (e.g., of h)) can be performed using any of the flow modalities previously described for the optional washing step (e.g., of b)).

In examples of the further method, the measuring methods (e.g., of i)) can comprise optical imaging and/or electronic interrogation methods, as previously described for such steps (e.g., of c)). The measurement methods may further comprise a transmembrane pressure-dependent modality. Such measurements can be performed as a single instance or as replicate instances, and reported as a single datum or as mean data.

In addition to the measurement methods (e.g., of c)) disclosed herein, a first analytical assay is used to determine the physical characteristics of the retained components. The first analytical assay is selected from infrared spectroscopy and Raman spectroscopy. Other, optional, additional assays may also be performed. For example, following either c), f) or i), electron microscopy and electron dispersive spectroscopy (EDS) and/or electron energy loss spectroscopy (EELS) may additionally be used to determine additional size and compositional characteristics of the retained components. In some examples, a physical property of a particular component type may be used for its characterization. In addition to the measurement methods (e.g., of c)) disclosed herein, a first analytical assay selected from infrared spectroscopy and Raman spectroscopy may be used to determine the physical characteristics of the retained components. For example, following either c), f) or i), electron microscopy and electron dispersive spectroscopy (EDS) and/or electron energy loss spectroscopy (EELS) may additionally be used to determine additional size and compositional characteristics of the retained components. In some examples, a physical property of a particular component type may be used for its characterization. As described above, a fluorescent signal of a cadmium selenide quantum dot, generated at a specified excitation wavelength and recorded at a specified emission wavelength, may additionally be used to confirm that the retained components are cadmium selenide quantum dots.

As a further example, following either c), f) or i), EDS or EELS may be used to determine additional size and/or composition of the retained components. The elemental and/or molecular compositional data derived from EDS or EELS may provide details on the type of retained component (e.g., the elemental or molecular composition is consistent with a plastic, a polymer, a metal, et cetera). The extent or size of such EDS or EELS spectra may further provide size of the retained component. Such EDS or EELS measurements may be combined with one or more of the optical modalities described herein. For example, optical microscopy may be used to image the size of retained components and EDS or EELS used to gather compositional measurements on the retained components. An algorithm may be used to automate the collection of optical and EDS or EELS measurements. For example, EDS or EELS is used to identify retained components of a certain composition (e.g., polymeric particles), such particles registered (e.g., positionally marked) on the microslit filter, and then optical microscopy used to size the registered and compositionally analyzed/identified particles. In various examples, the EDS or EELS and optical microscopy may be performed one after the other (in any temporal order) or may be performed simultaneously. In one example, the EDS or EELS and optical microscopy methods and related algorithms may be used as a test for polymeric particle (e.g., microplastic) concentration in a sample (e.g., any of the biological or non-biological samples disclosed herein, including a water sample), wherein micro plastic particles are enumerated (e.g., where their concentration is determined) in the sample by any of the other methods of the present disclosure. In another example, the EDS or EELS and optical microscopy methods and related algorithms may be used to evaluate the biological components produced in a cell culture, wherein the concentration of such biological components is determined) in the sample by any of the other methods of the present disclosure, and the EDS or EELS measurements are used to measure the size and/or composition of such biological components. The EDS or EELS and optical microscopy methods may be used for one, two, or more distinct component populations that have been captured and retained by one, two, or more microslit filters.

In a further example, as described above, a fluorescent signal of a cadmium selenide quantum dot, generated at a specified excitation wavelength and recorded at a specified emission wavelength, may be used to confirm that the retained components are cadmium selenide quantum dots. As additional examples of analytical assays that may be applicable to biological components of interest, following c), f) or i), electron microscopy may be used to characterize the size and/or ultrastructure of the retained components. As described above, the hemoglobin content within retained erythrocytes may be used as the basis of an absorbance optical signal at a specified wavelength for confirming that the captured components are erythrocytes. These analytical assay methods may be used for one, two, or more distinct component populations that have been captured and retained by one, two, or more microslit filters.

The first analytical assay is selected from Fourier-transformed infrared (FTIR) spectroscopy or Raman spectroscopy that are used to determine the size and/or composition of the retained microplastic components. For purposes of this disclosure, FTIR and Raman spectroscopy may be considered an optical modality or method. As an example, following either c), f) or i), FTIR and/or Raman spectroscopy may be used to determine additional size and/or composition of the retained components. The elemental and/or molecular compositional data derived from the FTIR or Raman measurements may provide details on the type of retained component (e.g., the elemental or molecular composition is consistent with a plastic, a polymer, a metal, et cetera). The extent or size of such FTIR or Raman measurements may further provide size of the retained component. Such FTIR and Raman microscopy or spectroscopy measurements may be combined with one or more of the optical modalities described herein. For example, optical microscopy may be used to image the size of retained components and FTIR or Raman microscopy used to gather compositional measurements on the retained components. An algorithm may be used to automate the collection of optical and FTIR or Raman microscopic measurements. For example, FTIR or Raman microscopy is used to identify retained components of a certain composition (e.g., polymeric particles), such particles registered (e.g., positionally marked) on the microslit filter, and then optical microscopy used to size the registered and compositionally analyzed/identified particles. In various examples, the FTIR or Raman microscopy and optical microscopy may be performed one after the other (in any temporal order) or may be performed simultaneously. In one example, the FTIR or Raman and optical microscopy methods and related algorithms may be used as a test for polymeric particle (e.g., microplastic) concentration in a sample (e.g., any of the biological or non-biological samples disclosed herein, including a water sample), wherein micro plastic particles are enumerated (e.g., where their concentration is determined) in the sample by any of the other methods of the present disclosure. In another example, the FTIR or Raman and optical microscopy methods and related algorithms may be used to evaluate the biological components produced in a cell culture, wherein the concentration of such biological components is determined) in the sample by any of the other methods of the present disclosure, and the FTIR or Raman measurements are used to measure the size and/or composition of such biological components. The FTIR or Raman and optical microscopy methods may be used for one, two, or more distinct component populations that have been captured and retained by one, two, or more microslit filters.

The components of interest are retained by the microslit filter during the first analytical assay. In various examples, the one or more analytical assays can be performed as either in situ or ex situ measurements, with respect to the fluidic devices used for the contacting and optional washing steps (e.g., steps a) and b), respectively). As an example of an in situ analytical assay, the microslit filter remains within a fully assembled or partially disassembled fluidic device when performing, for example, FTIR or Raman spectroscopy. In this example, the fluidic device may be transferred into an instrument for carrying out an analytical assay. In an example of an ex situ analytical assay, the microslit filter is extracted from a partially or fully disassembled fluidic device and the microslit filter is transferred to an instrument for carrying out, for example, FTIR or Raman spectroscopy or electron microscopy, EDS, or EELS. In this latter example, the extracted microslit filter may be optionally transferred to another substrate prior to transfer into the instrument carrying out an analytical assay. In another example, the step of enumerating the retained components (e.g., either step c), f) or i)) is performed as an in situ measurement that is then followed by an ex situ analytical assay.

In a further example of analytical assays, a retained component may be labeled with a unique binding agent, wherein each unique binding agent bears an unique enzyme, chromophore, fluorophore or quantum dot that endows a distinct optical signal to each retained component, upon reaction with the appropriate reagents (in the case of enzymes) or upon illumination at the appropriate wavelength (in the case of chromophores, fluorophores, and quantum dots). These binding agents may be conjugated to an optical detection moiety, wherein these optical detection moieties may include, but are not limited to, a fluorophore, a chromophore, a fluorescent polymeric nanocomponent, a quantum dot, or an enzyme or other catalytic molecule which exhibits or participates in substrate reduction process (or processes), such that these conjugates possess or yield an emission, a chemiluminescent or absorbance signal at a defined wavelength or range thereof. Further, substrates for enzymatic or catalytic reduction, as well as any required co-reagents for such reduction, may be added sequentially or may be concurrently added with binding agents. These analytical assay methods may be used for one, two, or more distinct component populations that have been captured and retained by one, two, or more microslit filters. Of course, other analytical assay methods may be used and these examples have been provided merely for exemplary purposes.

As a further method, an optional elution step may follow c), f) or i), for purposes of carrying out a second analytical assay on eluted components. For example, following c), f) or i), retained components can be transferred to another appropriate receptacle (e.g., vessel, surface, instrument, or the like) for subsequent second analytical assays. Elution of retained components may be performed by introducing a bolus of buffer solution to flush any retained components from the microslit filter by reversing any of the flow modalities used for initial retention of such complexes (e.g., during a)). Second analytical assays include, but are not limited to, assays well-known to those skilled in the related art, and may include, but are not limited to, a sequencing reaction, an amplification reaction, polymerase chain reaction, reverse transcriptase-polymerase chain reaction, ligase chain reaction, Northern blotting, Southern blotting, fluorescent hybridization, enzymatic treatment, labeling with second binding agents, enzyme-linked immunosorbent assay, immunocytochemistry, Western blotting, immunoprecipitation, fluorescence-activated sorting, optical imaging, electron microscopy, surface plasmon resonance, Raman spectroscopy, mass spectroscopy, Fourier-transformed infrared spectroscopy, or interferometry, among other possibilities. In some examples, the assay can be nanopore-based resistive pulse sensing; for example, as disclosed in Huff et al. (WO2016161402A1). In other examples, the assay can be arrayed imaging reflectometry (for example, as disclosed in Miller and Rothberg, U.S. Pat. No. 7,292,349). If multiple components of interest have been retained, then assays for multiplex detection may be used to analyze these two or more components. Of course, other possibilities exist and these examples have been provided merely for exemplary purposes.

In an example of the methods disclosed herein, the liquid sample is any one of the sample types disclosed herein and three microslit filters (as independent or combined configuration) are specified such that the first microslit filter captures and retains large polymeric particles (e.g., ≥ 20 µm diameter microplastic particles), the second microslit filter captures and retains medium polymeric particles (e.g., 10 µm to 20 µm diameter microplastic particles), and the third microslit filter captures and retains small polymeric particles (e.g., ≤ 10 µm diameter microplastic particles). In other examples, various combinations of microslit filters captures and retains distinct sets of polymeric particles from a sample. Of course, any combination of microslit filter opening sizes may be used in order to accomplish the desired capture and retention of particular populations of polymeric particles. These examples wherein sets of particle populations from a sample are enumerated (e.g., where their concentration is determined) may be useful as a test for microplastic particle contents in the sample and thus may be referred to as "a microplastic particle test" throughout the present disclosure. As examples, a microplastic particle test may be useful for determining the presence or absence of such polymeric particles in water samples drawn, for example, from a tap in a home or other building, in the effluent from a industrial process or a water sanitation process, from a water treatment process, among other possibilities. Similarly, such methods may be used to enumerate polymeric particle content with a food sample (e.g., milk, juice, beer, wine, among other possibilities). Any of these samples may be diluted prior to contact with the various microslit filters, and such dilution accounted when algorithms of the present disclosure calculate the particle number per volume passed through a fluidic device. The foregoing examples may be combined with one or more analytical assays (e.g., FTIR or Raman spectroscopy, electron microscopy, optical microscopy, among others) to determine additional size and compositional measurements on the polymeric particles. Each particle type may be measured as a single instance or as replicate instances, and the number of particles reported as a single datum or mean data.

In various examples, the methods of the present disclosure may comprise using a filter comprising one or more or a plurality of microslits and/or one or more or a plurality of nanopores. The method may further comprise enumerating one or more components retained on the filter comprising one or more or a plurality of microslits and/or one or more or a plurality of nanopores.

In various examples, a method comprises contacting a liquid sample with a mircoslit filter (e.g., filtering with a microslit filter), followed by contacting the liquid sample with a nanoporous filter. The method may further comprise enumerating one or more components retained on the microslit filter and/or nanoporous filter.

Also provided is a device comprising at least one microslit filter, wherein each microslit filter comprises a plurality of openings configured such that one or more microplastic components of interest are retained by the microslit filter and undesired components permeate through the microslit filter,
wherein the device further comprises a light source and a detector configured to record optical signals of an optical modality,
wherein the light source and the detector are configured to record optical signals of an optical modality by performing Fourier-transformed infrared spectroscopy or Raman spectroscopy on microplastic components disposed on the microslit filter;
wherein the microslit filter comprises a silicon nitride membrane and wherein in the case of infrared spectroscopy the microslit filter comprises an infrared reflective layer comprising metal selected from Au, Pt, Ag, Ir, Rh and combinations thereof or in the case of Raman spectroscopy the microslit filter comprises a Raman-silent layer comprising MgF₂ or CaF₂.

In various examples, the fluidic device comprises two or more independent microslit filters. In another example, the fluidic device comprises at least one microslit filter with two or more microslit filter elements, wherein the microslit filter element comprises microslit filter aspects within one substrate.

In various examples, a device of the present disclosure may comprise one or more microslit filter(s), each comprising a plurality of microslits, and one or more nanoporous filter(s), each comprising a plurality of nanopores. In various other examples, a device of the present disclosure may comprise a filter having one or more or a plurality of microslits and/or one or more or a plurality of nanopores. Such devices are suitable for use in methods of the present disclosure.

In an example, the present disclosure provides a fluidic device comprising at least one microslit filter with a membrane thickness of ≤ 500 nm and membrane apertures (e.g., openings) with an aspect ratio of ≥ 1:100, at least one light source, and at least one detector. In another example, the present disclosure provides a fluidic device comprising at least one microslit filter with a membrane thickness of ≤ 500 nm and membrane apertures with an aspect ratio of ≥ 1:100, and at least one transmembrane pressure sensor.

In a further aspect, the thickness (e.g., membrane thickness), porosity, and opening aspect ratio of microslit filters are specified in examples disclosed herein, such that the thickness and porosity properties promote low species permeation resistance and high permeability (e.g., high permeation capacity, low pressure operation), while the opening aspect ratio promotes non-fouling behavior and precision molecular cut-off (e.g., selective retention of components and selective permeation of other components). In further examples, the characteristics of microslit filters are specified for performing an upstream sample preparation. In various examples, the fluidic devices may be constructed such that the incorporated microslit filters can be optionally extracted from the fluidic device (upon partial or complete disassembly of the fluidic device) for carrying out the various methods of the present disclosure.

In an example of the present disclosure, a device for capturing and retaining a component of interest from a liquid sample comprises a fluidic device comprising one or more microslit filters or microslit filter elements, having at least one chamber or channel in fluidic contact with the cis-side of the microslit filter and at least one chamber or channel in fluidic contact with the trans-side of the microslit filter, wherein the cis and trans-sides oppose each other, and the cis- and trans-side chambers or channels are fluidically connected by a plurality of openings in the one or more microslit filters.

The microslit filters of the fluidic device have specified physical characteristics to promote high cis-to-trans permeation of liquid samples and to promote non-fouling behavior and precision molecular cut-off. The microslit filters comprise a plurality of arrayed openings that fluidically connect/communicate with their opposing cis- and trans-sides. The microslit filter can have a range of membrane thickness; for example, the membrane thickness can be from 50 nm to 25 µm, including all values to the nm or µm and ranges therebetween. In another example, the membrane thickness can be from 50 nm to 1 µm, including all values to the nm or µm and ranges therebetween. In another example, the membrane thickness can be from 50 nm to 500 nm, including all values to the nm and ranges therebetween. The microslit filters can have a range of porosity; for example, the porosity can be from less than 1 % to 75 %, including all integer % values and ranges therebetween. In another example, the porosity can be from 5% to 30%, including all integer % values and ranges therebetween. The microslit filter can have a range of aspect ratio for its openings; for example, its openings can be cubic prisms, rectangular prisms, or trapezoids and be 0.5 µm to 15 µm in width and 5 µm to 100 µm in length, including all values to the µm and ranges therebetween thus possessing a range of aspect ratio (in terms of width to length) between 1:0.33 to 1:200. In a particular example, the microslit filter is 400 nm thick, has approximately 17% porosity, and has openings of 9 µm width and 50 µm length, and an aspect ratio of 1:5.5. In another particular example, the microslit filter is 400 nm thick, has approximately 9% porosity, and has openings of 1 µm width and 50 µm length, and an aspect ratio of 1:50. Of course, other values are possible and these are merely listed as examples. In another particular example, the microslit filter (e.g., the membrane) is 400 nm thick, has approximately 11% porosity, and has openings of 0.5 µm width and 50 µm length, and an aspect ratio of 1:100. In another particular example, the microslit filter (e.g., the membrane) is 200 nm thick, has approximately 30% porosity, and has openings of 0.2 µm width and 10 µm length, and an aspect ratio of 1:50.

The microslit filters of the fluidic devices should be further specified so that contacting the liquid sample performs the desired filtration steps of the present disclosure. Accordingly, the microslit filters of the fluidic device should be specified in terms of the width of their openings for selective capture and retention of components and for selective permeation of other components. Further, the relative size of components to be retained in an upstream sample preparation should be considered with respect to the opening width of the microslit filter used for such purposes.

The fluidic device may accomplish the filtration (e.g., of a)) using several flow modalities and filtration system configurations. In an example, the fluidic device uses dead-end filtration, wherein contact with the microslit filter involves flow that is normal to the microslit filter surface. The liquid sample is introduced to the cis-side of the filter and either hydrostatic pressure or the application of cis-side positive pressure or trans-side negative pressure initiates flux from the cis- to the trans-side of the filter. Positive and negative pressures may be generated by, for example, gravity/hydrostatic pressure or by pumping, vacuum, gas pressurization, centripetal force and the like. In various examples, the fluidic device is a stirred cell dead-end filtration system that uses gas pressurization or vacuum, or the fluidic device is a centrifuge insert dead-end filtration system that uses centripetal force. In another example, the fluidic device uses transmembrane pressure differential and tangential flow, wherein contact with the microslit filter involves flow that is tangential to the microslit filter surface, the biofluid or the liquid sample is introduced to the cis-side of the filter, bulk flow is initiated on both cis- and trans-sides of the filter such that a transmembrane pressure is generated (e.g., relative negative pressure on the trans-filter side), thus initiating flux from the cis- to the trans-side of the filter. The relative bulk flow rate on the cis-filter side should be greater than the bulk flow rate on the trans-filter side in order to create the desired transmembrane pressure vector. These tangential flow and transmembrane flow vectors create diafiltration as described previously herein. In various examples, the cis- and trans-sides of the microslit filters are fluidically connected to chambers or channels of these opposing sides and bulk flow is initiated in these chambers or channels using gas pressurization or pumping apparatus (e.g., a syringe or peristaltic pump, or the like). In various examples, the fluidic device is a tangential flow filtration system.

The fluidic device of the present disclosure can further comprise two or more independent microslit filters in a variety of configurations. In one example, a liquid sample is passed from the cis-side of a first microslit filter to its trans-side, and then from the trans-side of a first microslit filter to the cis-side of a second microslit filter, and then to the trans-side of a second microslit filter. In another example, a liquid sample is passed from the cis-side of a first microslit filter to the cis-side of a second microslit filter. As another example, a liquid sample is passed from the cis-side of a first filtration membrane of a first microslit filter to the trans-side of a first filtration membrane and into a first intervening fluidic cavity, then is passed from the first intervening fluidic cavity to the cis-side of a second filtration membrane of a first microslit filter and then to the trans-side of the second filtration membrane. Then, the liquid sample is passed from the cis-side of a first filtration membrane of a second microslit filter to the trans-side of a first filtration membrane and into a second intervening fluidic cavity, then is passed from the second intervening fluidic cavity to the cis-side of a second filtration membrane of a second microslit filter to the trans-side of the second filtration membrane of the second microslit filter, and so on. Thus, the two or more independent microslit filters can be disposed in different fluidic planes (e.g., in series) or disposed on the same fluidic plane (e.g., in parallel). In various examples, the independent microslit filters can be connected by tubing or channels with inlets and outlets between the two or more microslit filters. As an example, a fluidic device incorporating at least two independent microslit filters may be used to perform an upstream sample preparation and component enumeration method of the present disclosure. As another example a fluidic device incorporating at least three independent microslit filters may be used to perform a method of the present disclosure (e.g., a complete blood cell count, a microplastic test, or a biological component production test). Of course, other fluidic device configurations are possible and these examples have been provided merely for exemplary purposes.

In another example, the fluidic devices further comprise one or more microslit filters with two or more filtration membranes (e.g., microslit filter elements). In one example, the fluidic device comprises a microslit filter with multiple filtration membranes disposed on a combined substrate. As one example using a microslit filter with multiple filtration membranes in a combined substrate configuration, a liquid sample is initially contacted by a first microslit filtration membrane, the fluid is then passed to and contacted by a second filtration membrane, and then the fluid is passed to and contacted by any third and/or successive filtration membranes. In this example, the successive and multiple filtration membranes (e.g., microslit filter elements) are within one combined substrate and are disposed along the length of one channel, such that the fluid is successively contacted by each element as it passes through the channel and thus the elements are all of the same fluidic plane. Such substrates are fabricated with the various microslit filter elements (e.g., filtration membranes) on distinct regions of one substrate. As one example, a fluidic device incorporating a combined microslit filter with at least three filtration membranes (or elements) may be used to perform a complete blood cell count method of the present disclosure. Of course, other fluidic device configurations are possible and these examples have been provided merely for exemplary purposes.

The fluidic devices may accomplish any optional washing steps (following the filtration steps) using similar flow modalities and filtration system configurations as those used for filtration steps. For example, in a stirred cell filtration system or a centrifuge tube insert filtration system (both of which are configured for dead-end/normal flow), one or more bolus of fresh buffer may be introduced to the cis-side of the microslit filter (following the filtration step), and either hydrostatic pressure or the application of cis-side positive pressure or trans-side negative pressure initiates flux of the buffer. In an additional example, in a tangential flow filtration system (configured for tangential flow), one or more bolus of fresh buffer may be introduced to the cis-side of the microslit filter (following the filtration steps), and bulk flow on the cis-side used to initiate buffer flow. Application of transmembrane pressure would be optional in this latter example.

The fluidic devices may accomplish any optional elution steps using similar flow modalities and filtration system configurations as those used for filtration steps. However, in some examples, the fluidic devices should be able to operate the flow modality in reverse of that initially used for the filtration steps. As one example, the retained components may be eluted from the cis-side of a microslit filter by introduction of a bolus of buffer to the cis-side of the contacting microslit filter, resuspension of the components by any mechanical mixing method, and the re-suspended components removed from the fluidic device (e.g., by a manual or automated pipet). As another example, the pressurization within a stirred cell filtration system may be reversed, following an introduction of fresh buffer to the cis-side of the contacting microslit filter, and the pressurization used to flush off retained components into the buffer. As yet another example, the relative bulk flow rates and direction of the transmembrane pressure vector may be reversed in a tangential flow system to flush off retained components into a flow of fresh buffer introduce to the cis-side of the contacting microslit filter.

The various configurations of fluidic devices comprising two or more microslit filters may use tangential flow alone or tangential flow in combination with normal flow. As an example, tangential flow alone can be used to contact a liquid sample by the cis-sides of successively configured microslit filters in either the independent or combined configurations if these elements are all of the same plane. As a different example, tangential flow can be used to contact a liquid sample by the cis-sides of two successively configured microslit filters (these two elements being of the same plane), and then normal flow used to pass the resultant permeate of the second microslit filter to the cis-side of a third microslit filter (the second and third microslit filters being of different planes). The fluid can be successively transferred between elements either as continuous flow (passing fluid through all elements in uninterrupted succession) or as discrete flow (passing fluid one element at a time with interrupted flow at each element). Tangential and normal flow within these exemplary fluidic devices would be initiated by the same bulk flow modalities described above. Accordingly, the further examples of the fluidic device can be a tangential flow filtration system or a combined tangential/normal flow filtration system. The microslit filter comprises a silicon nitride membrane and in the case of infrared spectroscopy the microslit filter comprises an infrared reflective layer comprising metal selected from Au, Pt, Ag, Ir, Rh and combinations thereof, and in the case of Raman spectroscopy the microslit filter comprises a Raman-silent layer comprising MgF₂ or CaF₂.

For example, these filters and membranes may comprise a suspended membrane layer that was fabricated by: 1) patterning and etching the openings into silicon nitride, using well-known photolithography and reactive ion etching methods, followed by etching to suspend the silicon nitride through a substrate (e.g., a silicon wafer); 2) embossing openings into polyurethane or poly-dimethyl-siloxane, using a master mould with a negative relief pattern of the openings; 3) solvent-casted polymer membranes of appropriate thickness and opening size and aspect ratio; 4) track-etched polymer membranes of appropriate thickness and opening size and aspect ratio; 5) patterned photoresist membranes (e.g., SU-8) with openings of appropriate size and aspect ratio that are fabricated by well-known photolithography methods (e.g., patterning SU-8 photoresist on a Si wafer and subsequent lift-off of the SU-8 membrane after its patterning); or 6) stainless steel, nickel or other alloy membrane with electro-formed openings of appropriate size and aspect ratio. In a further example, the microslit filter, with at least one first filtration membrane, at least one intervening fluidic cavity, and at least one second filtration membrane, is fabricated by methods disclosed in Striemer et al. U.S. Pat. No. 62/248,467, wherein microslit filters may be fabricated by transmembrane etching through their openings (e.g., as openings in a silicon nitride layer disposed onto a Si wafer substrate). Of course, other microslit filter fabrication methods are possible and these examples have been provided merely for exemplary purposes.

The microslit filters of the fluidic devices have a specified thickness so that the fluidic device operates at a range of low pressurization when performing the filtration steps; e.g., 10 Pa to 1.0 kPa, and all Pa values therebetween.

Microslit filters are incorporated into fluidic devices as independent or combined elements for carrying out the methods of the disclosure. For example, one or more microslit filters, each comprising a suspended silicon nitride membrane on a silicon substrate, can be fabricated using a Si wafer and the resultant microslit filter(s) incorporated into a fluidic device. As another example, two or more aspects of a Si wafer can be fabricated to yield two or more microslit filter elements (e.g., aspects) and the resultant microslit filter with two or more elements incorporated into a fluidic device. The suspended silicon nitride membranes correspond to freestanding regions wherein the plurality of openings through the membranes (e.g., cubic or rectangular prisms) fluidically connect cis- and trans-sides of the membranes.

Although the various examples disclosed herein use microslit filters in preferred embodiments and examples, other types of silicon-based membranes or filters could be used in the methods of the present disclosure. For example, the membrane could be a nanoporous silicon nitride membrane (NPN). Examples of NPN membranes and the fabrication of such membranes are disclosed in U.S. Patent No. 9,789,239 (Striemer et al. "Nanoporous Silicon Nitride Membranes, and Methods for Making and Using Such Membranes"). In another example the membrane could be a microporous silicon nitride membrane (MP SiN). Examples of MP SiN membranes and the fabrication of such membranes are known in the related art. In yet another example, the membrane could be a microporous flat tensile silicon oxide membrane (MP SiO2). Examples of MP SiO2 membranes and the fabrication of such membranes are disclosed in U.S. Patent No. 9,945,030 (Striemer et al. "Free-Standing Silicon Oxide Membranes, and Methods of Making and Using Same").

The microslit filters can be functionalized with moieties that decrease the adhesion to liquid sample constituents (e.g., coatings to reduce or prevent fouling). For example, a silanization process may be used for functionalizing microslit filters, wherein a vapor phase silane source contacts the filters or membranes, the silane reacts with a functional surface group of the filters or membranes, and the silane is further derivitized with a poly-ethylene glycol moiety of 5-10 carbon atoms in length. As another example, the microslit filters may be functionalized with a carbinylation process as disclosed in Shestopalov et al. (U.S. Pat. No. 9,089,819), the disclosure of which is hereby incorporated in its entirety by way of reference), wherein a vapor phase carbine source (e.g., a diazirine compound) contacts the filters, the carbine reacts with a functional surface group of the filters or membranes (e.g., an aliphatic monolayer), and the carbine is further derivitized with a poly-ethylene glycol moiety of 5-10 carbon atoms in length. In a further example, the microslit filter is functionalized using a epihalohydrin-based process as disclosed in Carter and Roussie U.S. Provisional Pat. Appln. No. 62/614,232, wherein silicon nitride microslit filters are chemically treated, reacted with gas-phase epichlorohydrin, and then terminally reacted with an amine-containing reactant (e.g., amino-PEG or ethanolamine). In these examples, the terminal surface hydroxyl groups provide a non-fouling surface (e.g., a non-fouling surface treatment). Furthermore, such hydroxylated surfaces may improve eluting of retained components, if their elution is desired. Alternatively, these functionalization processes may be used for functionalizing the surfaces of microslit filters to promote their selective interactions with certain components. For example, a silane, carbene, or epihalohydrin-treated microslit filter may be further functionalized with one or more binding agents disclosed herein to promote capture and retention of components bearing target ligands for such binding agents. As another example, the capture and retention of negatively charged components at neutral pH (e.g., silica) may be improved if microslit filters where functionalized with terminal moieties that endowed them with positive charge at neutral pH (e.g., a moiety with a primary amine of pKa in the range of 7-8). As another example, surface functionalization may be used to promote the permeation of undesired components likely to be present in a liquid sample (e.g., by repelling the undesired components by a charged surface of similar polarity). Of course, other possible surface functionalization possibilities exist and these examples have been provided merely for exemplary purposes.

The fluidic device may further comprise a light source and a detector for recording optical signals of any first and/or second analytical assay. The light source may be a laser, a photodiode array, or any similar light source. The detector may be a spectrophotometer, a photometer, a leumeter, a charge-coupled device, or any other similar device. The light source and detector may be appropriate for carrying out the optical imaging, optical diffraction, and optical-dependent second analytical assays or modalities, either for determining their number or other characterization or compositional analyses. For example, the light source and the detector would be configured as required by the optical imaging or optical diffraction methods and used to read optical signals generated at the microslit filters; for instance, the microslit filter or sorting membrane is disposed between the light source and the detector.

The fluidic device may further comprise an algorithm for calculating the concentration of components in a liquid sample, from a known or measured volume of liquid sample passed in the fluidic device and from the determined number of components of interest captured and retained on the microslit filter, and may further account for any dilution of the liquid sample (if applicable). The fluidic device may further comprise a flow meter for measuring the volume of liquid sample passed in the fluidic device. In an example, the fluidic device comprises an algorithm for calculating component concentration. In another example, the fluidic device comprises a flow meter for measuring volume passed of the liquid sample.

In an example, a fluidic device for optical imaging and/or optical diffraction modalities for determining the number of retained components of interest comprises at least one sample microslit filter, at least one reference microslit filter, a light source, and a detector, and can carry out the methods of the optical imaging and optical diffraction modalities, as well as optical-dependent second analytical assays. In some examples, the sample and reference microslit filters are disposed between the light source and the detector, such that the microslit filters can be trans-illuminated by the light sources and their resultant imaging or diffraction spectra recorded by the detector. In a further example, a fluidic device for such optical modalities may further comprise an additional microslit filter, such that the additional microslit filter performs sample preparation prior to the optical modality methods. Such fluidic devices may further comprise a signal processing algorithm, wherein the signal processing algorithm automates the collection and comparison of sample and reference microslit filter imaging and/or diffraction spectra. In some examples, multiple fluidic devices of the optical modalities may be configured such that multiple components of interest (from one liquid sample) can be analyzed on multiple sample microslit filters in parallel.

The fluidic device may further comprise one or more pairs of electrodes, a voltmeter, and/or a function generator for carrying out an electronic interrogation modality based on transmembrane electrical resistance or impedimetric spectroscopy.

In an example, a fluidic device for electronic interrogations for determining the number of retained components of interest comprises at least one sample microslit filter, at least one reference microslit filter, a voltmeter, and at least one pair of electrodes. In other examples, the fluidic device may further comprise a function generator. Such fluidic devices can carry out the electronic interrogation methods of the present disclosure. In some examples, the sample and reference microslit filters are disposed between the one or more pairs of electrodes, such that the transmembrane electrical resistance and/or impedance recorded by a detector. In a further example, a fluidic device for electronic interrogations may further comprise an additional microslit filter, such that the additional microslit filter performs sample preparation prior to the electronic interrogations. Such fluidic devices may further comprise a signal processing algorithm, wherein the signal processing algorithm automates the collection and comparison of sample and reference microslit filter transmembrane electrical resistance and/or impedance. In some examples, multiple fluidic devices of the electronic interrogation may be configured such that multiple components of interest (from one liquid sample) can be analyzed on multiple sample microslit filters in parallel.

In an example, a fluidic device for pressure-dependent modalities for determining the number of retained components of interest comprises at least one sample microslit filter, at least one reference microslit filter and one or more pressure sensors (e.g., transmembrane pressure sensor), and can carry out the methods of pressure-dependent modalities. In some examples, the sample and reference microslit filters are disposed between the one or more pressure sensors, such that the microslit filters' transmembrane pressure (e.g., fluidic resistance) can be measured. In a further example, a fluidic device for such pressure-dependent modalities may further comprise an additional microslit filter, such that the additional microslit filter performs sample preparation prior to the transmembrane pressure measurements. These fluidic devices may further comprise a signal processing algorithm, wherein the signal processing algorithm automates the collection and comparison of sample and reference microslit filter transmembrane pressure. In some examples, multiple fluidic devices of the pressure-dependent modalities may be configured such that multiple components of interest (from one liquid sample).

In an aspect outside the scope of the claims, the present disclosure provides a kit comprising one or more specified devices and, optionally, one or more reagents for carrying out the methods of the disclosure, wherein the physical properties of microslit filters (e.g., thickness, porosity and opening aspect ratio and size) are specified relative to the size of components of interest. Accordingly, the devices and reagents of the disclosed kit are intended to be used as a combined system.

In an example, a kit for capturing and retaining components of interest from a liquid sample comprises one or more fluidic devices, wherein the fluidic device of the kit comprises one or more microslit filters or more microslit filters with one or more filter elements. The width of the microslit filters' openings are specified relative to the size of the components of interest Cath are to be captured and retained by the one or more microslit filters (e.g., the components' diameter is greater than the width of the microslit filters' openings). The kit comprises elements to perform one or more method of the present disclosure.

In an example, a kit of the present disclosure further comprises one or more pump, one or more flow meter, one or more centrifuge, and one or more algorithm for component concentration calculation, for carrying out any of the flow modalities of the present disclosure.

In an example, a kit of the present disclosure further comprises one or more algorithm for component size and/or composition determinations.

In an example, the kit of the present disclosure may further comprise one or more pairs of electrodes, one or more voltmeter, and one or more function generators, for carrying out the electronic interrogation methods of the present disclosure.

In an example, the kit of the present disclosure may further comprise one or more pressure sensors (e.g., transmembrane pressure sensor) for carrying out the transmembrane pressure measurements of the present disclosure.

In a further example, a further kit comprises one or more buffer, one or more wash or elution solution, one or more binding agent, a light source, and a detector. The one or more binding agents comprise affinity moieties that bind ligands of the retained components on microslit filters. The light source and the detector of the kit comprise elements for carrying out first and/or second analytical assays on any retained components retained on microslit filters. The one or more buffer, washing, and/or elution solutions comprise reagent solutions for carrying out any optional washing or elution steps. In further examples, a further kit of the present disclosure comprises plasmonically active microslit filters and thermal elements to specify temperature during optical imaging.

In a further example, a further kit comprises one or more additional microslit filters for performing upstream sample preparation.

The fluidic device of the optical diffraction modalities kit may further comprise at least one sample microslit filter, at least one reference microslit filter, a light source, and a detector. The light source, detector, and signal processing algorithm of the kit comprise elements for trans-illumination of sample and reference microslit filters and for recording, collecting, and comparing their respective optical images and/or diffraction spectra.

In a further example, a kit of the present disclosure may further comprise one or more light source, a detector and an algorithm, for carrying out Raman microscopy and optical microscopy for determining the size and/or composition of components of a sample retained by microslit filters.

The steps of the method described in the various embodiments and examples disclosed herein are sufficient to carry out the methods of the present disclosure. Thus, in an embodiment, a method consists essentially of a combination of the steps of one or more of the methods disclosed herein. In another embodiment, a method consists of such steps.

The following examples are presented to illustrate the present disclosure. They are not intended to be limiting in any matter. The invention is defined by the appended claims.

### EXAMPLE 1 (reference only)

This example provides a description of examples of devices and methods of the present disclosure, as well as an exemplary method and use of the present disclosure for retaining and measuring a biological component from a biological liquid sample to perform a blood cell count.

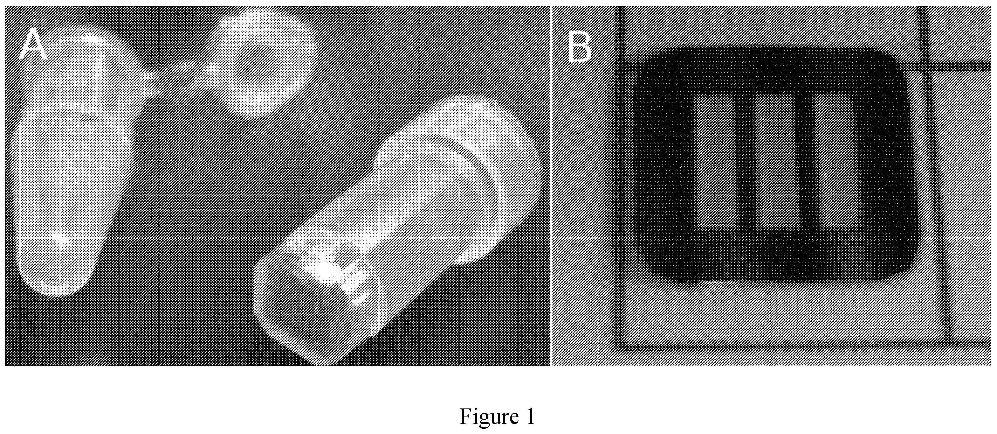

Figure 1A shows a representative fluidic device incorporating a microslit filter, wherein the microslit filter is integrated into a centrifuge tube insert fluidic device for dead-end (normal) flow filtration purposes. Figure 1B shows a representative microslit filter comprising 400 nm thick silicon nitride membranes, with three 0.7x3 mm suspended membranes, disposed on a silicon substrate of 5.4x5.4 mm and 0.3 mm thickness. The three 0.7x3 mm silicon nitride membranes further comprise a plurality of 8x50 µm openings patterned and etched through the 400 nm thick silicon nitride membranes. Conventional photolithography, reactive ion etching, and wet chemistry through-wafer etching were used to fabricate such microslit filters.

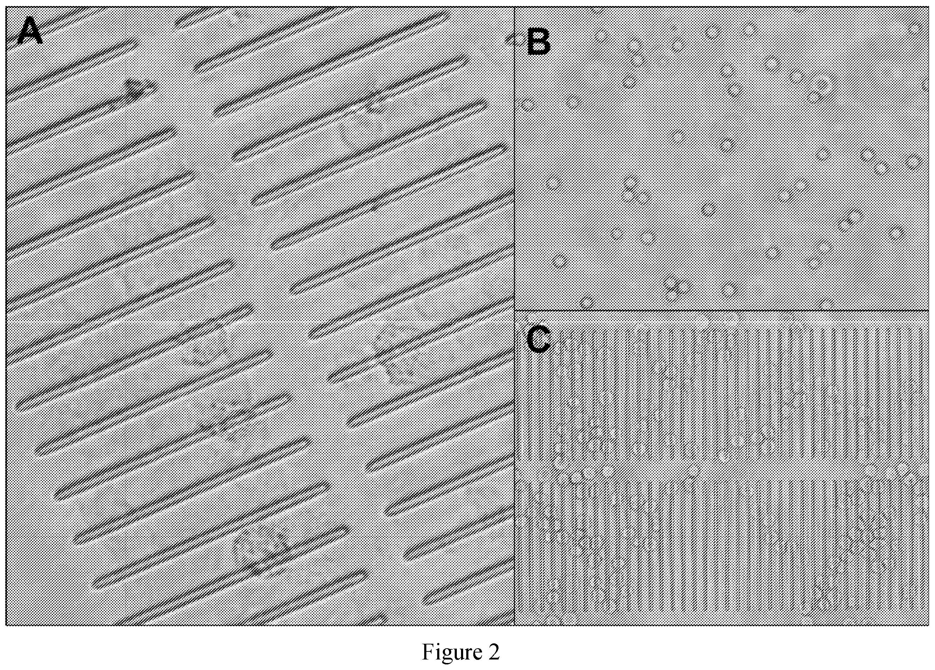

Figure 2A shows a similar microslit filter as shown in Figure 1B, after contact with a 0.1 mL solution of a biological liquid sample, comprising whole blood diluted to 0.1% (v/v) in phosphate-buffered saline (PBS) formulated at 270 mOsm, pH 7.4. Such a dilution represents one of the optional pre-treatment steps of the present disclosure. The biological components (e.g., cells) retained by this microslit filter are consistent with white blood cells (e.g., leukocytes). The fluidic device used for this filtration comprised a microslit filter similar to that shown in Figure 1A in all respects except that the microslit filter of Figure 2A comprised membranes with 1x50 µm openings. Figure 2B shows a second filter, comprising nanoporous silicon nitride (100 nm thickness, 60 nm average diameter pores, 20% porosity), after contact with a 0.1 mL volume of the flow-through from the filtration performed using the 1x50 µm microslit filter of Figure 2A. The cells retained on the nanoporous filter from the flow-through are consistent with red blood cells (e.g., erythrocytes). Figure 2C shows a third filter after contact with a 0.1 mL volume of the flow-through from the filtration performed using the 1x50 µm microslit filter of Figure 2A. The cells retained on this third filter are consistent with red blood cells (e.g., erythrocytes). The fluidic device used for this filtration comprised a microslit filter similar to that shown in Figure 1A in all respects except that the microslit filter of Figure 2C comprised membranes with 0.5x50 µm openings. All filtrations shown in Figures 2A-C were done by centrifugation at 600xG for 10 minutes, then the microslit filters were extracted from fluidic devices (e.g., for an ex situ measurement), and imaged on a confocal light microscope at 40X magnification.

### EXAMPLE 2

This example provides a description of the optical diffraction modality of the present disclosure for a model non-biological component retained and measured from a liquid sample.

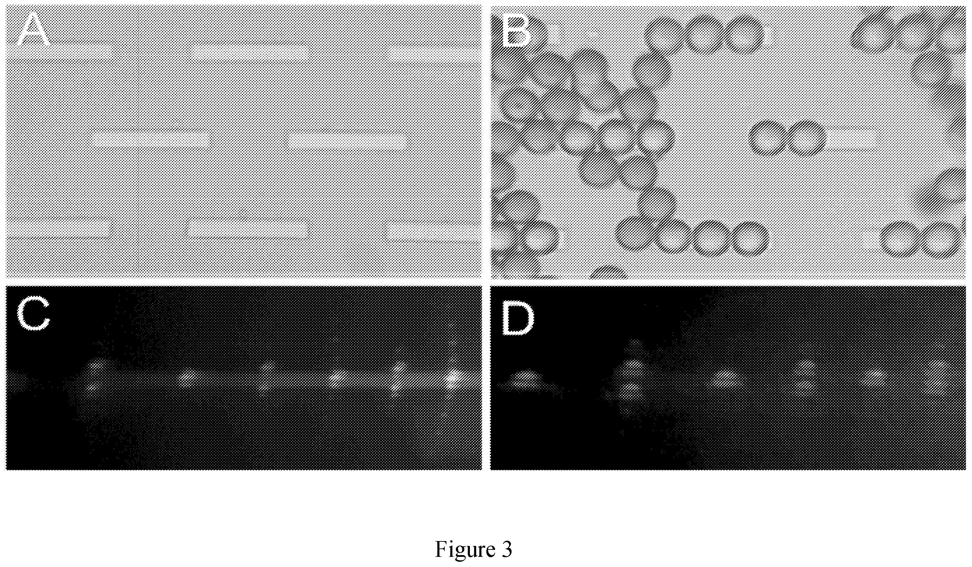

Figure 3A shows the native surface of the microslit filter of Figure 1B via light microscopy). Figure 3B demonstrates an identical filter retaining 16 µm polystyrene beads. Figures 3C, and 3D demonstrate the corresponding diffraction patterns of the two former microslit filters, where Figure 3C is the reference image for the diffraction pattern shift observed by microslit occlusion in Figure 3D.

### EXAMPLE 3

This example provides a description of the microslit filter-fluidic cavity monolithic structure of the present disclosure.

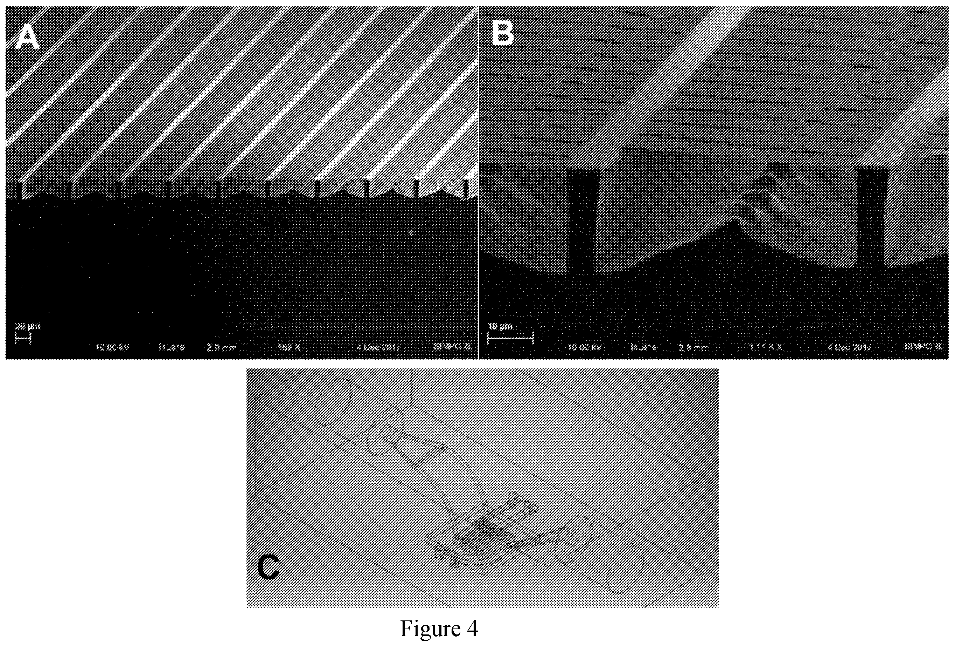

Figure 4A shows an electron microscopy image of an intervening fluidic cavity fabricated by transmembrane etching through microslit openings. Fluidic connection to this intervening fluidic cavity is through the microslit openings, within the resultant filtration membranes. Figure 4B shows one potential scheme for fluidically connecting multiple filtration membranes and intervening fluidic cavities.

Figure 4C shows a fluidic rendering of a device for fluidically addressing the membrane system from Figures 4A and 4B. The plurality of fluidic cavities is interconnected in series by a gasket interface.

### EXAMPLE 4

This example provides a description of a tangential flow fluidic device incorporating microslit filters.

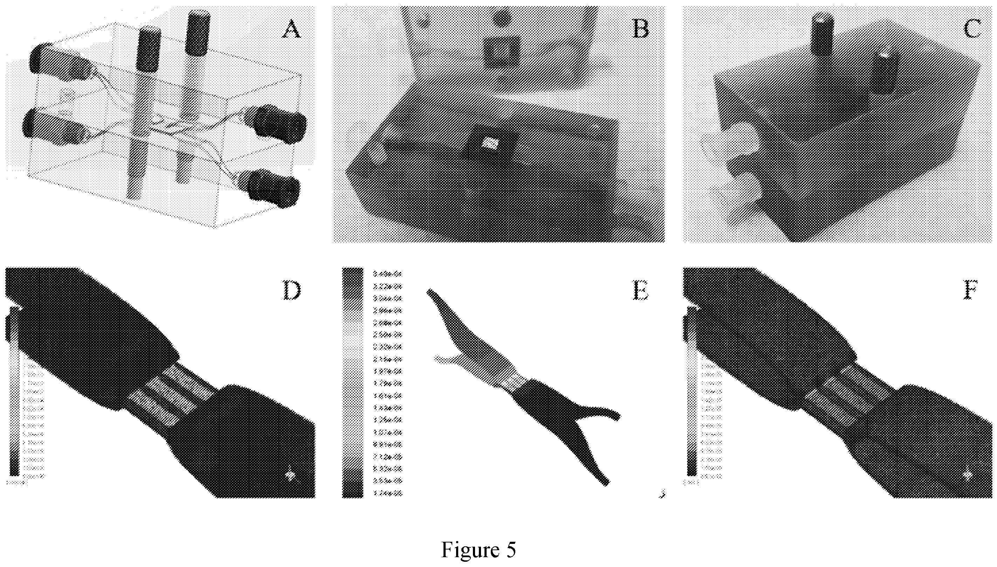

Figure 5 shows a panel of images describing the design and modeling of an exemplary tangential flow fluidic device for incorporating microslit filters (Figure 5A). A 3D-printed prototype with polycarbonate body and elastomer gasket (dark center region) is shown after partially assembly of the device with a microslit filter installed in the centering gasket (Figure 5B), and after complete assembly (Figure 5C). Using ANSYS software, surface velocity (Figure 5D), system pressure (Figure 5E), and shear stress (Figure 5F), were modeled to verify suitability for utilizing whole blood in the device.

This figure demonstrates the critical importance of membrane thickness on operating pressurization. As this simulation shows, the transmembrane pressure differential (TMP) increases proportionally to the membrane thickness. In this simulation, the TMP of a representative 50 nm thick membrane is very low (~12 Pa). As the membrane thickness increases to 5 µm and then 50 µm, there is a significant increase in TMP from ~20 Pa to ~65 Pa, respectively. Thus, low membrane thickness (e.g., ≤ 1 µm thick) enables far lower operating pressures relative to thicker membranes (e.g., ≥ 5 µm thick).

### EXAMPLE 5 (reference only)

This example provides a description of the pressurization within an exemplary tangential flow fluidic device incorporating microslit filters.

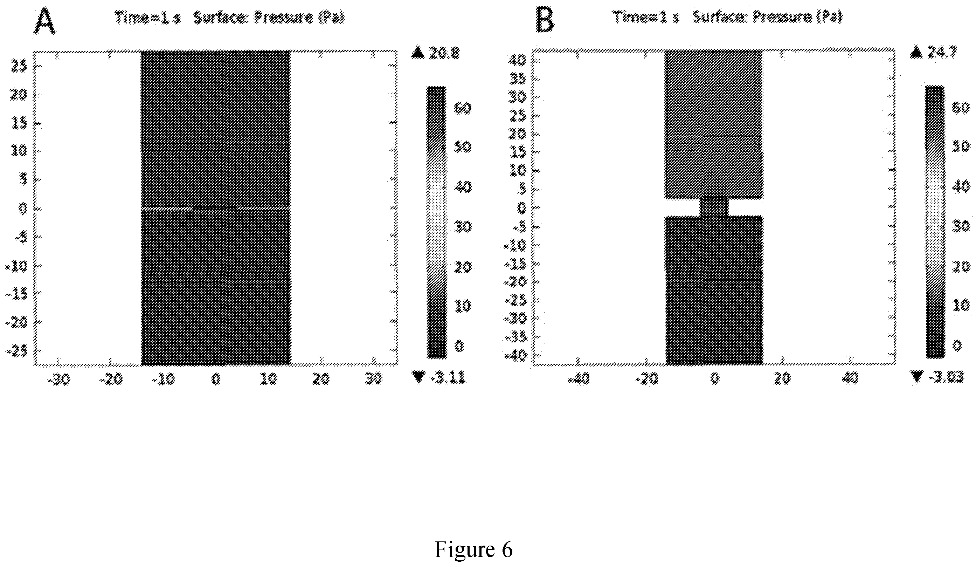

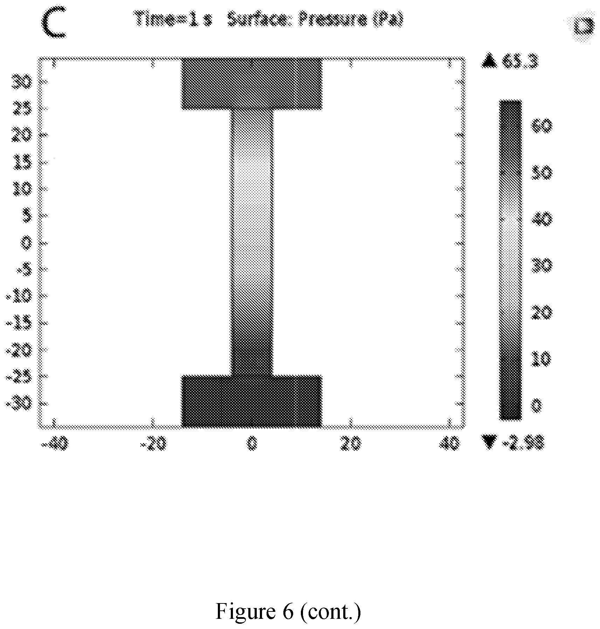

Figure 6A-C demonstrate the results of a pressure drop analysis which evaluated microslit height (e.g., thickness of a filtration membrane) for a suspension of cells to minimize sheer stress and subsequent cell damage. Figure 6A demonstrates a negligible 20.8 Pa pressure drop for the exemplary 400nm thick microslit filters described in the present disclosure. Increasing membrane thickness to 5 and 50 µm respectively (Figures 6B and 6C) yields a concomitant decrease in pressure through the membrane of 25 Pa and 65 Pa respectively.

### EXAMPLE 6

This example provides a description of a microplastic water test by one exemplary method of the present disclosure.

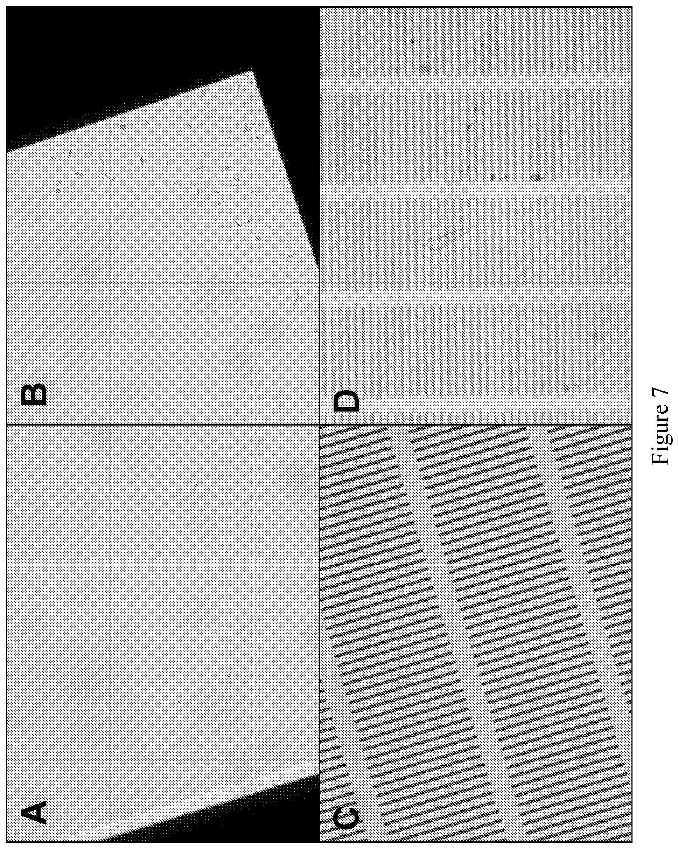

Figure 7 shows the resulting phase contrast microscopy images taken at 40x magnification of the resultant membrane filters after filtering two different water samples. The fluidic devices where similar to those shown and used in Examples 1-2. In one case, centrifuge spin columns incorporating nanoporous silicon nitride (NPN) filters with 50 nm average pore diameter were used to retain particles from either (A) MilliQ water (18.3 mOhm resistivity, 0.2 µm filtered), or (B) Tap water with no pretreatment. The water samples were centrifuged at 1,000xG for 10 minutes and 2 mL water samples were passed through each fluidic device. Microslit filter-incorporating fluidic devices were used to retain particles from either (C) MilliQ water (18.3 mOhm resistivity, 0.2 µm filtered), or (D) Tap water with no pretreatment. The water samples were centrifuged at 1,000xG for 10 minute and 2 mL water samples were passed through each fluidic device. After centrifugation, the membranes from all devices were extracted (e.g., for an ex situ measurement) and imaged via phase contrast light microscopy (all images 40X). Retained particles are consistent with both inorganic and organic composition, based on observed morphology.

### EXAMPLE 7

This example provides a description of a method and use of the present disclosure for optional elution following selective retention of a model non-biological component from a liquid sample.

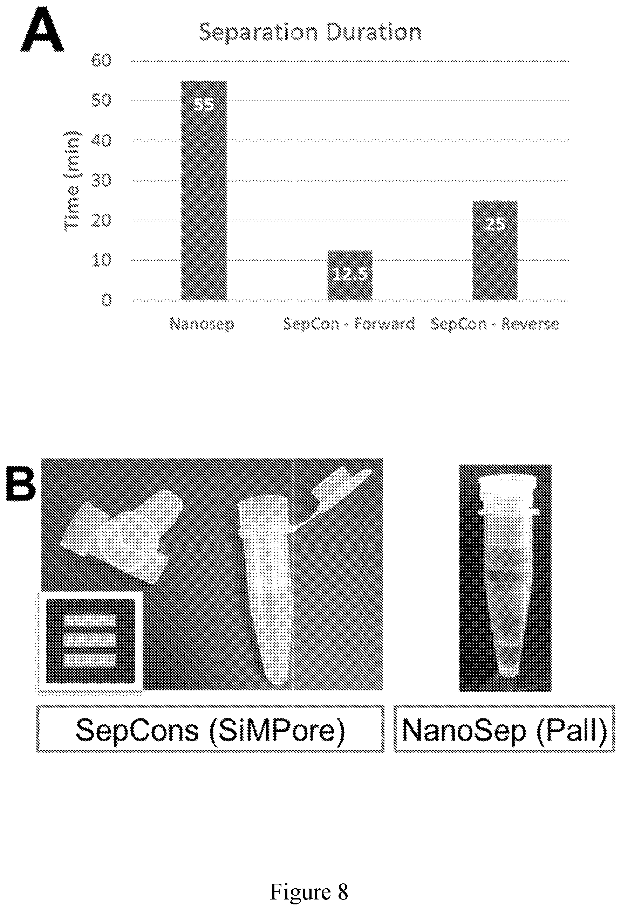

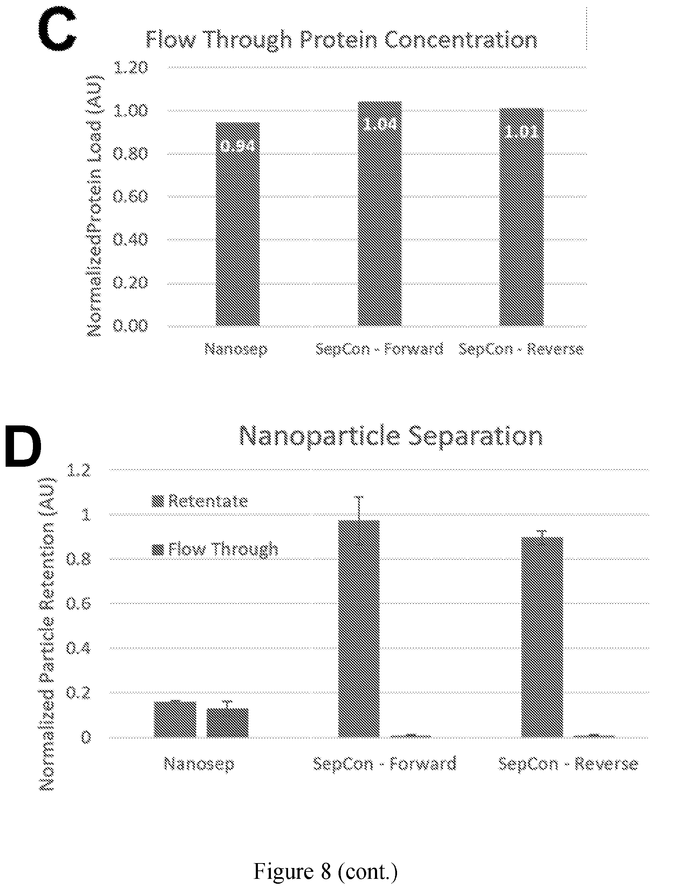

Figure 8 shows the results from the selective retention by a microslit filter of a polymeric particle. A suspension of fluorescent red polystyrene particles (2.0 µm average diameter) was prepared at 1x10^6 particles/mL in PBS with 0.5 mg/mL bovine serum albumin (BSA). 400 µL of this solution was centrifuged through various fluidic devices until the volume was passed and the time to complete volume passage was recorded. Fluidic devices with 1x50 slit openings were used (Figure 8B; labeled as "SepCon"), wherein one set of fluidic devices operated in the "Forward" mode similar to this shown in Figures 1-2, while a second set of fluidic devices operated in a "Reverse" mode, wherein the flow moves in an opposite flow pattern to that in the Forward mode. The same particle solution was also centrifuged through a commercial polymeric filter spin column (Pall NanoSep^{®}, 300 kDa cut-off Omega membranes). Figure 8A demonstrates the higher permeability of microslit filters versus polymeric filters, as evidenced by the significantly reduced filtration time for the microslit filter-containing spin column to pass the entire 400 µL volume (12.5-25 minutes versus 50 minutes). Figure 8C demonstrates essentially complete recovery of the BSA in the filtrate produced by the microslit filter-containing spin columns in either Forward or Reverse modes, whereas 6% protein loss was observed to the polymeric filter. Figure 8D shows both higher particle retention and particle recovery (using an optional elution method of the present disclosure) for microslit filter-containing spin columns relative to the commercial polymeric filter spin column.

### EXAMPLE 8 (reference only)

This example provides a characterization of the filtration properties using model non-biological components for a representative nanoporous filter.

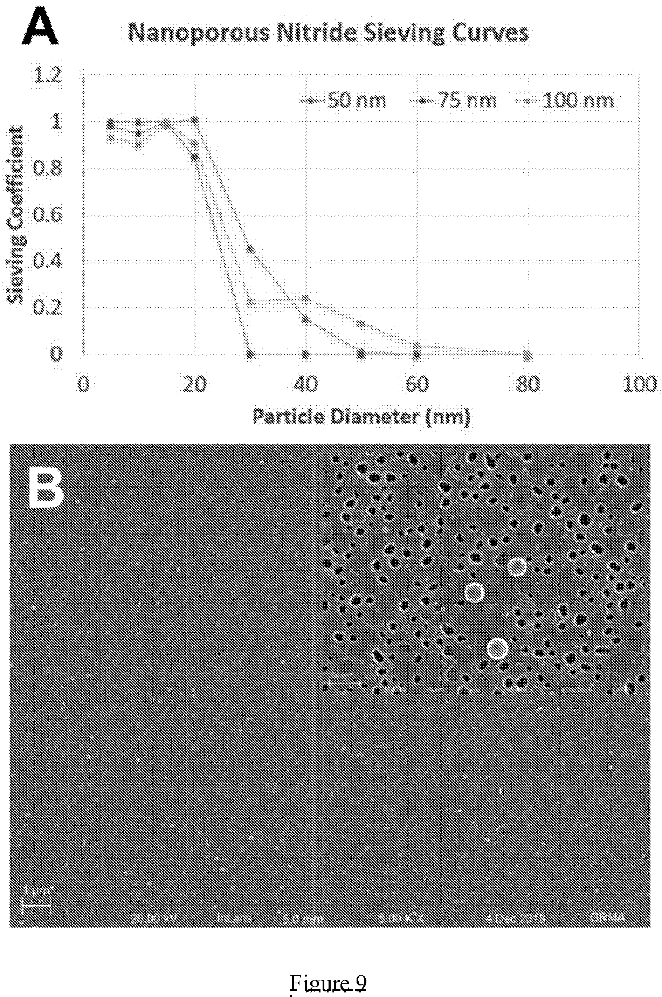

Figure 9A shows particle retention and permeation results for three different nanoporous filters (50 nm, 75 nm, and 100 nm thickness, each with its own corresponding average pore diameter (~23 nm, ~33 nm, and ~45 nm, respectively). For each data point in Figure 9A, a distinct population of gold nanoparticles (with distinct average diameter) were dissolved in water and then passed through each nanoporous filter via centrifugation. The amount of gold particles retained and permeated was measured and results are plotted as percent transmission (e.g., sieving coefficient). Figure 9B shows a representative 100 nm-thick nanoporous filter retaining 100 nm-average diameter polystyrene particles that was imaged by scanning electron microscopy following the filtration. The nanoporous filter was extracted from the spin column used for this filtration, thus this example demonstrates an ex situ analytical assay of the present disclosure.

### EXAMPLE 9

This example provides a characterization of the filtration properties using non-biological components for two representative microslit filters.

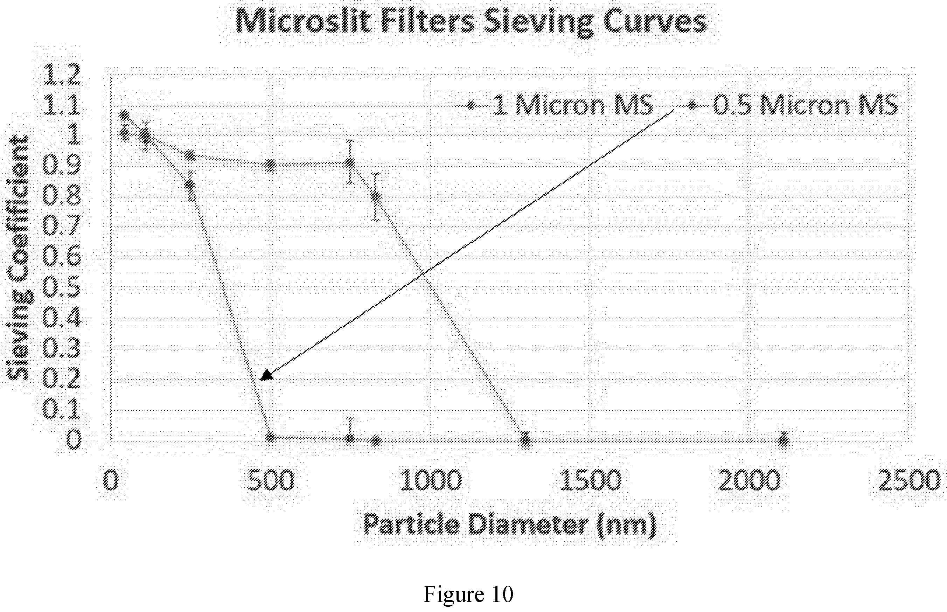

Figure 10 shows particle retention and permeation results for two different microslit filters with 0.5x50 µm and 1x50 µm slit openings (both with 400 nm thick silicon nitride membranes). For each data point shown in Figure 10, a distinct population of fluorescent polystyrene particles (with distinct average diameter) were dissolved in PBS to 1x10^6 particles/mL and then passed through each microslit filter via centrifugation. The amount of polystyrene particles retained and permeated was measured and results are plotted as percent transmission (e.g., sieving coefficient).

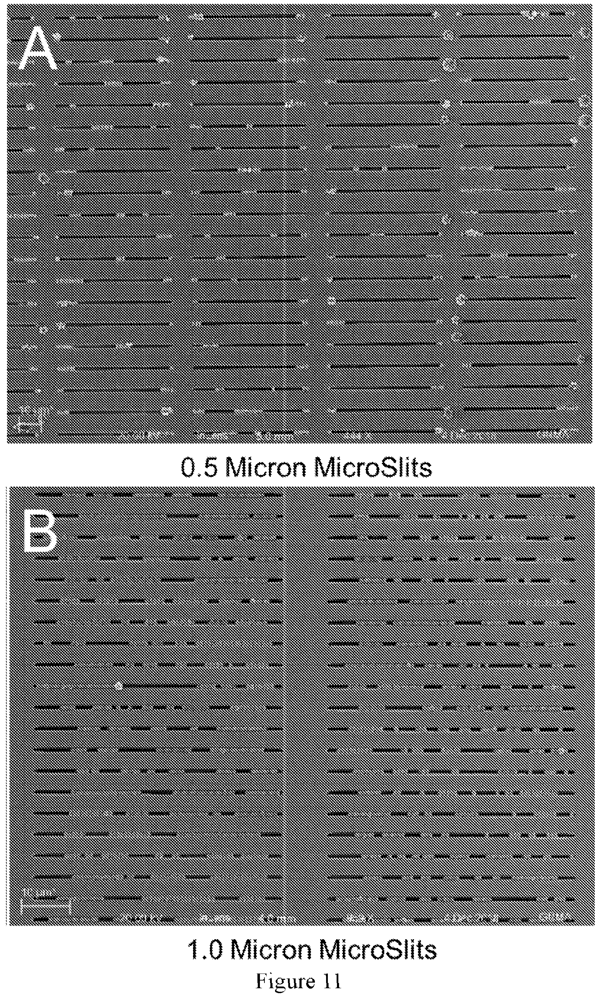

Representative 0.5x50 µm slit opening microslit filters (Figure 11A) and 1x50 µm slit opening Microslit filters (Figure 11B) are shown, as imaged by scanning electron microscopy following filtration of particles and extraction of the microslit filters from fluidic devices. Thus, this example represents an ex situ measurement. Solutions of either 850 nm or 1.25 µm diameter polystyrene particles dissolved in PBS were passed via centrifugation through either 0.5x50 µm or 1x50 µm slit opening microslit filters, respectively, prior to extraction and imaging.

### EXAMPLE 10

This example provides a description of an exemplary use of the transmembrane pressure-dependent method of the present disclosure using model non-biological components and a representative microslit filter.

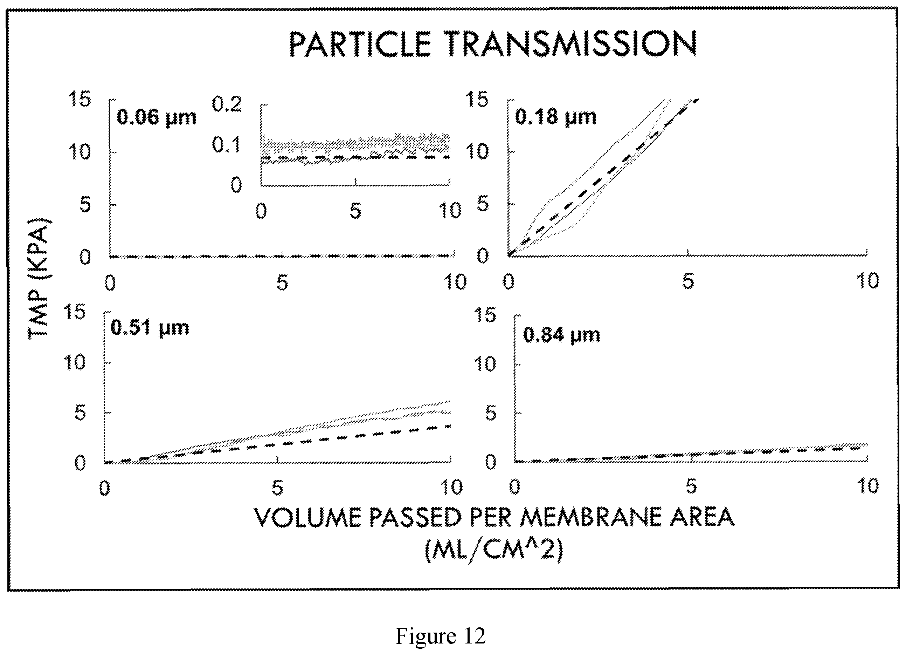

Figure 12 shows particle retention and permeation results for a microslit filter with 0.2x10 µm slit openings (200 nm thick silicon nitride membranes). For each set of data shown in Figure 12, a distinct population of fluorescent polystyrene particles (with distinct average diameter) were used. Particles were passed via normal flow filtration. The number of polystyrene particles retained and permeated was measured and results are plotted as percent transmission (e.g., sieving coefficient). Particles were diluted in 0.1 M carbonate buffer (pH 9.4) with 0.01% v/v Tween-20. The buffer was prepared with ultra-pure water and prefiltered through a 0.2 µm surfactant-free cellulose acetate syringe filter. All particles were diluted to a final concentration of 0.003% w/v and sonicated for 15 minutes in an ultrasonic bath immediately before use.

The particle filtration experiments were performed in a normal flow, constant flux configuration where TMP was monitored relative to atmospheric reference for all samples passed. The microslit filters were incorporated into spin columns (per Figures 1-2), which were connected to a PEEK adapter for flow and pressure recording. A USB output pressure transducer (Omega PX409) monitored changes in transmembrane pressure in real-time. Flow was supplied by a syringe pump (Harvard Apparatus PhD 2000); for all experiments a flux of 0.8 mL cm-2 min-1 was used. The transmembrane pressure rise was most pronounced as the particle size approached the narrow width of the microslit filters' openings.

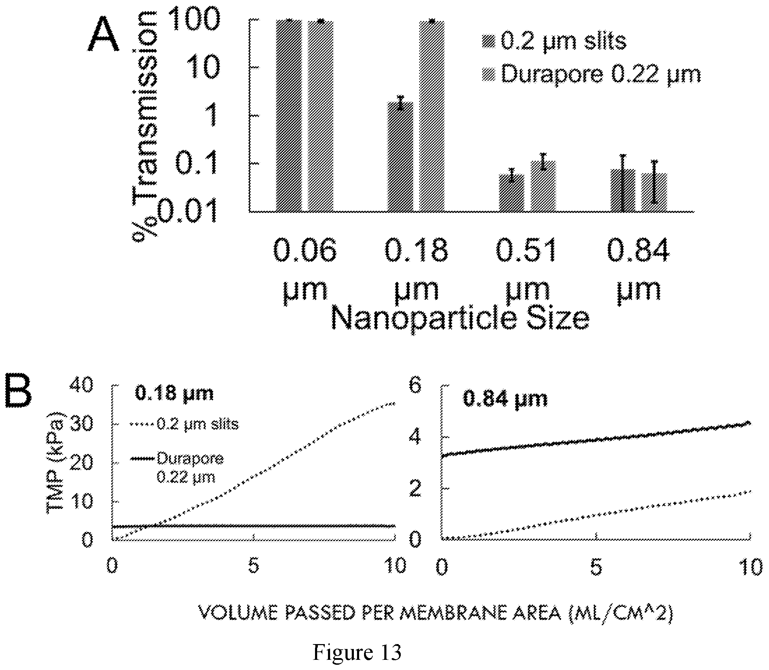

Figure 13 shows (A) transmission of various nanoparticle sizes through both the 0.2 µm slit membrane and a Durapore 0.22 µm PVDF membrane as a comparison. (B) Difference in fouling between the 0.2 µm slit membrane and the Durapore membrane. Lines shown are the average of 3 filtration runs.

### EXAMPLE 11

This example provides a description of an ex situ analytical assay of the present disclosure, with optical microscopy used to register components and infrared spectroscopy used to identify the composition of two types of model non-biological components (e.g., polymethylmethacrylate and polyethylene particles retained by microslit filters).

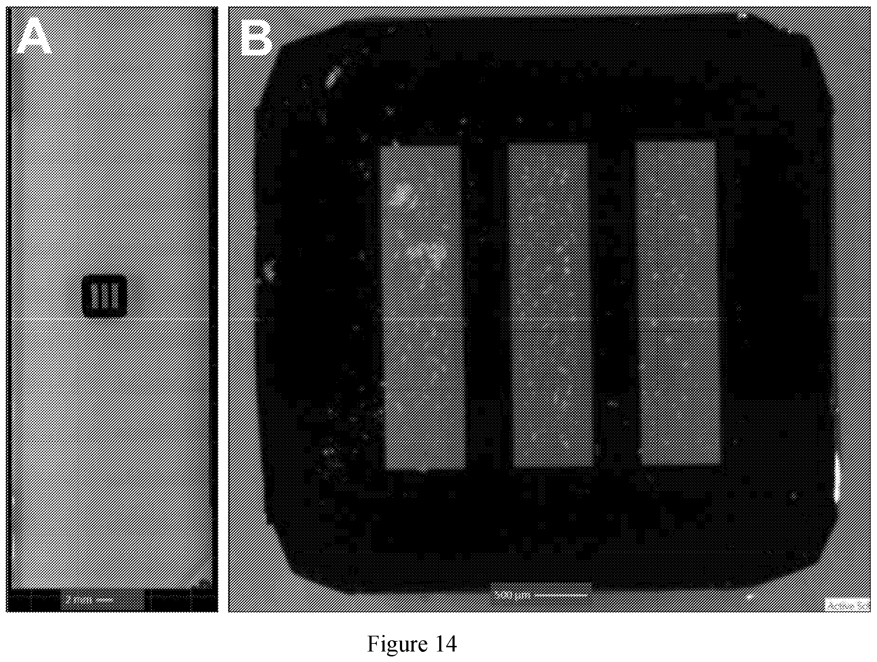

Figure 14 shows a 0.5x50 µm slit opening microslit filter used to filter a solution of polymeric particles that ranged in size from 1.0 to 15 µm diameter and comprising three distinct compositions (polystyrene, polyethylene, and polymethylmethacrylate). The particles were dissolved to a concentration of 1.0 µg/mL in PBS pH 7.4. Centrifugation was used (600 XG, 20 minutes) to filter 500 µL solution samples. An optional wash step was performed by passing 500 µL of MillliQ water through the spin columns (centrifuged as before). Each microslit filter was then extracted from the spin columns and potted onto MirrIR slides (aluminum coated glass slides; a.k.a. "Kelvey slides") using Norland 68, optical-grade, UV-cured adhesive. This potting process provided an infrared (IR) reflective background for subsequent analyses.

Figure 14A shows images of the microslit filter affixed to the MirrIR slide after Norland 68 bonding. Figure 14B shows a phase contrast light microscopy image of the Microslit filter in 14A at higher magnification, enabling resolution of the polymethylmethacrylate (PMMA) particles retained on the microslit filter.

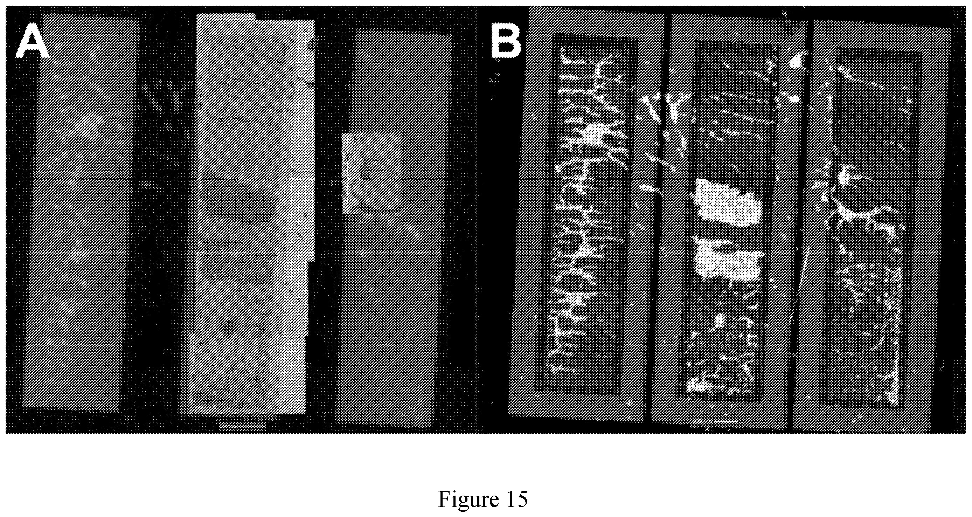

Figure 15A shows a phase contrast light microscopy image of a microslit filter with retained polyethylene (PE) particles. Figure 15B shows the the same region of interest as in Figure 15A, with equal magnification, using an Agilent 8700 LDIR at 1790 cm-1; 8 min, 1 µm pixel size. These data demonstrate a strong IR signature of the microplastic particles retained on the membrane surface relative to standard light microscopy.

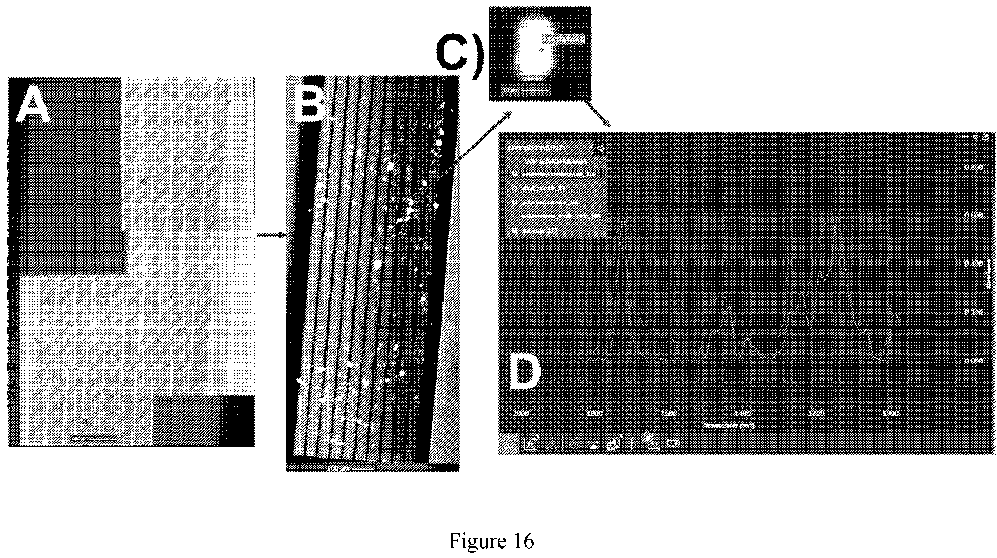

Figure 16 demonstrates an FTIR microscopy workflow for the identification and quantitation of unknown components (e.g. microplastic particles) captured by the microslit filter from a liquid sample (e.g., a water sample). A similar workflow could be used for another spectroscopy methods for the analytical assay (e.g., Raman or electron dispersive spectroscopy). Figure 16A shows a region-enhanced composite image of PMMA microparticles retained on a Microslit filter using phase contrast light microscopy. Figure 16B demonstrates an image of the same membrane and region of interest taken using imaging FTIR. Figure 16C demonstrates the automated identification of a plastic particle using an algorithm to register and then to identify the particle's composition (example shows a 10 µm diameter polymeric particle). The 8700 LDIR software demonstrating a 10-micron particle). Figure 16D shows the IR spectra of the 10 µm particle compared to a reference spectrum drawn from a reference library, showing that the particle's composition most closely matches that of PMMA.

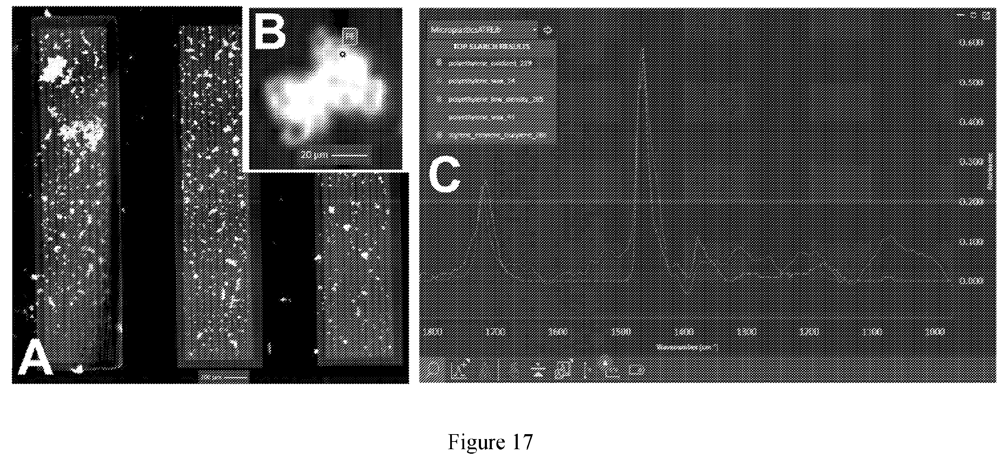

The example shown in Figure 17 is similar to that shown in the previous figure. Figure 17A shows a microslit filter with retained PE particles imaged using the 6700 LDIR system in IR mode. Figure 17B shows a ~24-micron condensate of multiple PE particles identified by the LDIR software. Figure 17C shows the best-fit spectra matching that of PE. These data indicate microplastics may be retained selectively by microslit filters, and so retained, can be sized and compositionally analyzed by analytical assays such as FTIR or other similar spectroscopic methods.

### EXAMPLE 12 (reference only)

This example provides a description of a representative biological component production test using the transmembrane pressure-dependent method of the present disclosure for two types of biological components (e.g., Adenovirus and Maraba virus) and compares the method between a representative microslit filter and a representative polymeric filter.

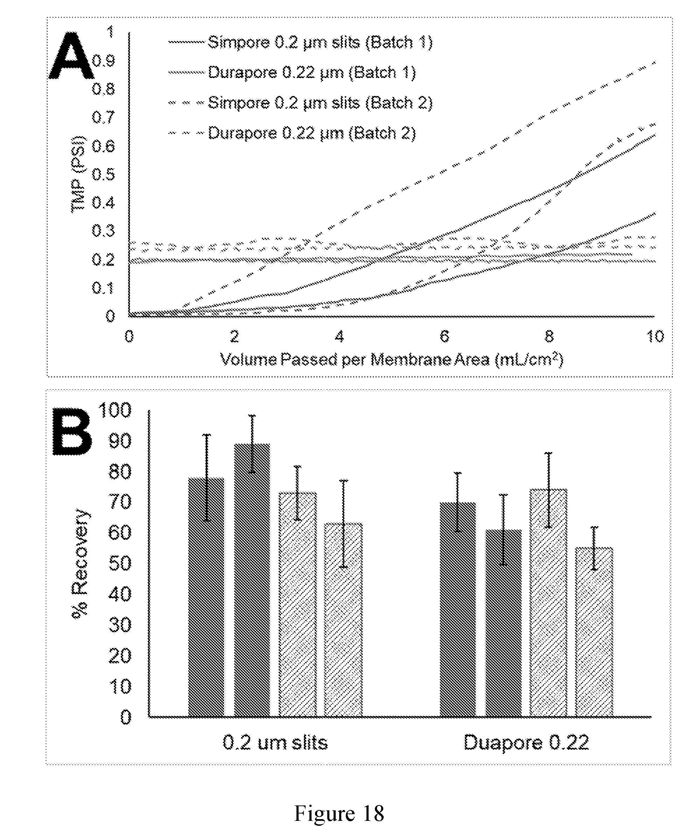

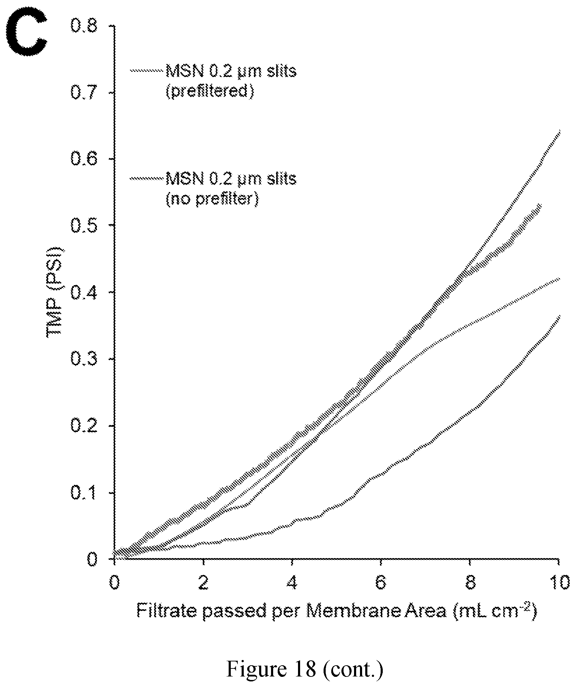

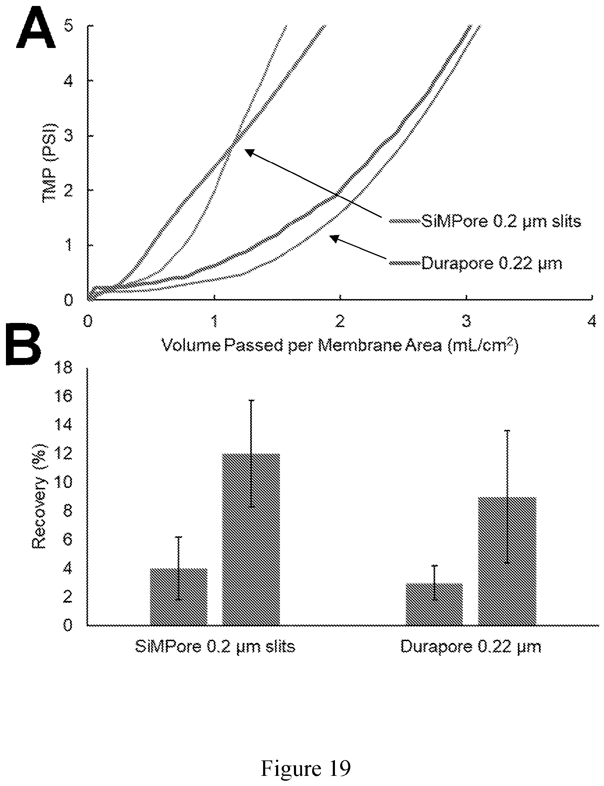

Figure 18 and 19 demonstrate filtration of a biological component produced for potential therapeutic applications (e.g., gene therapy, oncolytic viral therapy). Two different batches of adenovirus feedstock were prepared according to the method of Kawka et al (2019) Purification of therapeutic adenoviruses using laterally-fed membrane chromatography. J Memb Sci 579, 351-358. Viral suspension was tittered to therapeutic dosing equivalent by diluting either batch in PBS supplemented with BSA. Feedstocks from both batches were passed through 0.2x10 µm microslit filters.

Figure 18 compares the filtration of the two adenovirus feedstock preparations when filtered by either 0.2x10 µm microslit filters (Figure 18A) or conventional 0.2 µm pore size polymeric filters (Figure 18B; Millipore Durapore^{™} membranes). Filtration was assessed by the volume permeated and the transmembrane pressure (TMP) profile during filtration. The TMP profiles demonstrate that microslit filters are more sensitive to the size of components in the feedstock, as the faster rise in TMP for microslit filters suggests. A higher virus titer was recovered in the permeate of microslit filters, as measured by plaque forming unit (PFU) assay (Figure 18B). In all cases, two replicate experiments were performed for each filter type. Figure 18C shows a pre-treatment of the same Adenovirus feed stock as above using a 0.5x50 micron MicroSlit filter. Upon filtration 0.2X5 micron Microslit filter, the same TMP rise was observed, suggesting that the 0.5X50 micron Microslit filter did not retain any aggregates larger than 0.5 microns from the feedstock supply. These data imply that any aggregates within the feedstock may have an average particle diameter between 200 and 500 nm. This example further demonstrates a serial filtration as described in the present disclosure.

Figure 19 shows a similar experiment to that shown in the previous figure. In a similar manner to Figure 18, Figure 19A shows TMP rise relative to feedstock volume passed per cm^2 of filter surface area. In these experiments, the 0.2x10 µm microslit filters showed a rapid increase in TMP, indicating sensitive detection of components in the feedstock that were larger than the slit openings. Similar experiments were performed in parallel using 0.2 pore size Durapore ^{®} filters. Figure 19B shows higher recovery of virus in the permeate from microslit filters.

### EXAMPLE 13 (reference only)

This example provides a description of a representative biological component production test using the transmembrane pressure-dependent method of the present disclosure for a third type of a biological component (e.g., Brevumondias diminuta bacteria) and compares the method between a representative microslit filter and a representative polymeric filter.

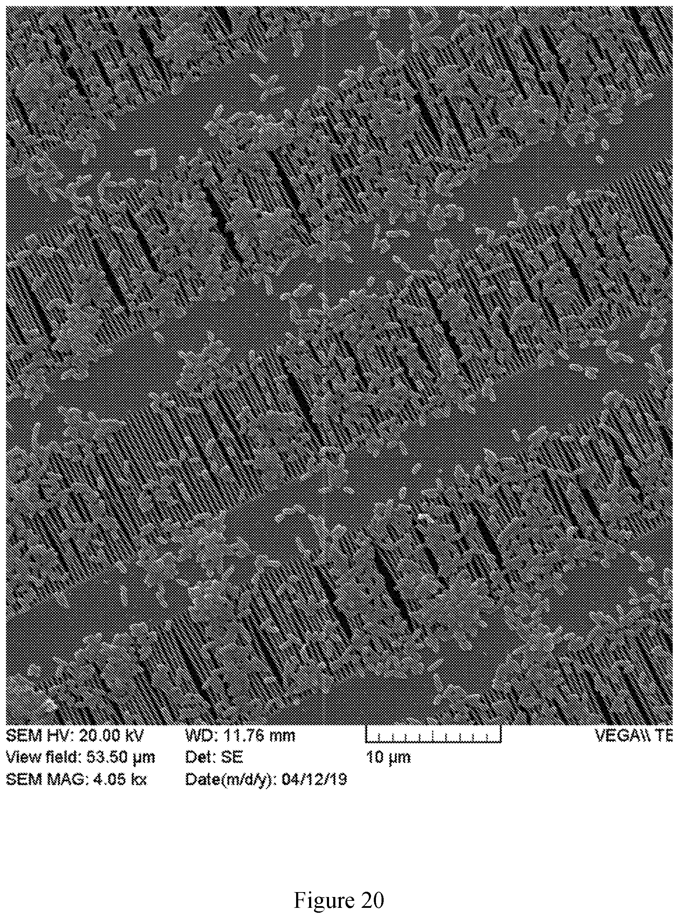

Figure 20 shows the resulting 0.2x10 microslit filter after filtration of a suspension solution of B. diminuta, using cryo-transmission electron microscopy. Thus, Figure 20 represents a further example of an ex situ analytical assay. In all related experiments, bacteria were grown on tryptic soy agar plates or as suspension cultures in saline lactose broth or tryptic soy broth. B. diminuta ATCC^{®} 19146 was used throughout. Following 24-hour cultures in tryptic north and saline lactose broth, material dilutions were prepared in saline lactose broth. The filtration apparatus shown in Figure 13 was used for the related experiments and volume passed and TMP were recorded. The apparatus was wetted with saline lactose broth and an aliquot of permeating broth was recovered for plate-culture assay. B. diminuta suspension was supplied at 2 mL cm-2 min-1. The filtrate was collected, streaked on plates, and cultured for 48 hours, after which plate colonies were counted. All experiments were performed in triplicate. These data show complete retention of bacteria. Moreover, the TMP profiles for 0.2x10 µm microslit filters show a dependence on bacterial concentration in the supplied feedstock, demonstrating that TMP can be used to evaluate the amount of biological components in a liquid sample.

### EXAMPLE 14

This example provides a fuller description of various membranes and filters represented throughout the present disclosure in the exemplary devices and methods.

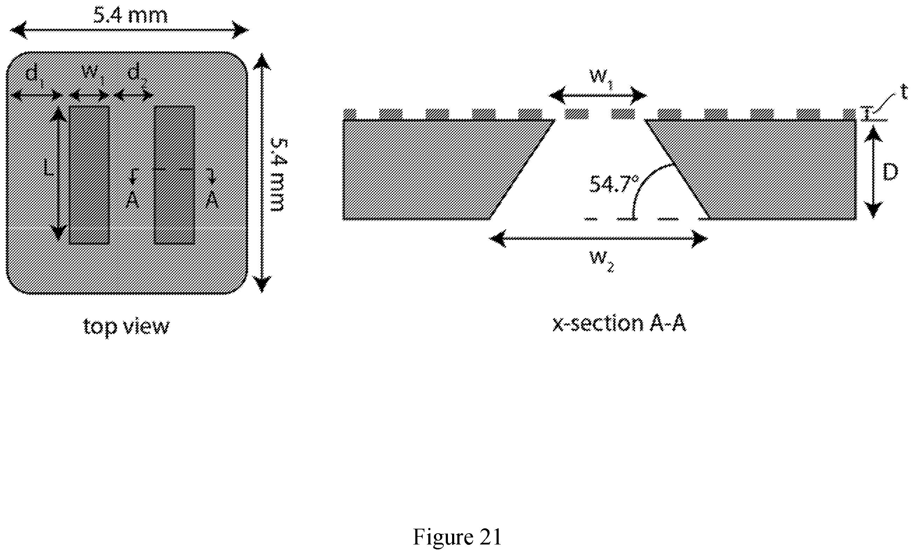

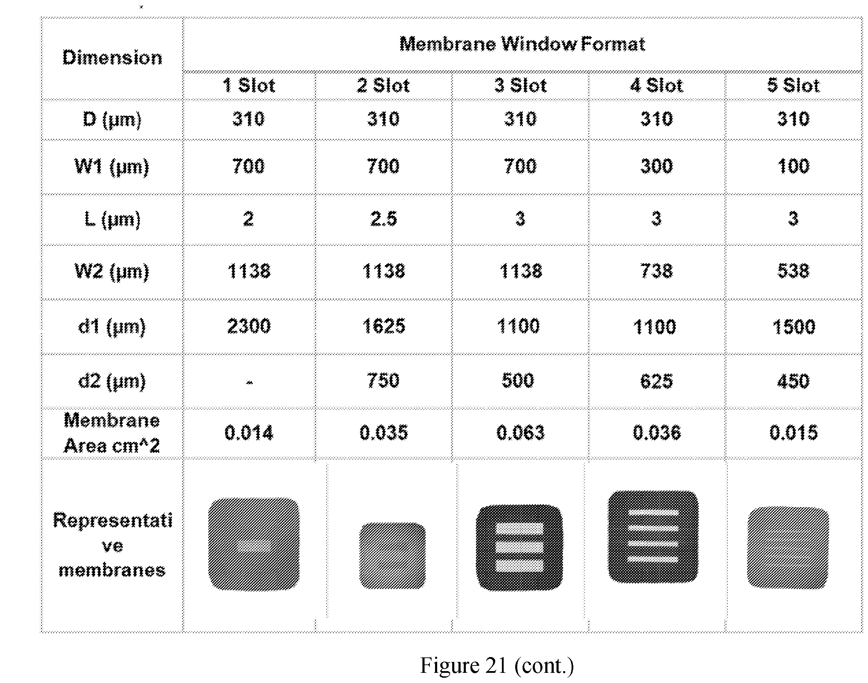

Figure 21 provides descriptive statistics for various membrane filters routinely produced by silicon-based fabrication processes, including the 5.4x5.4x0.3 mm format used throughout the present disclosure. This format is integrated into the centrifuge spin column fluidic devices; for example, those fluidic devices shown in Figure 1. Corresponding light microscopy images of the various membrane formats are also shown, with varying amounts of freestanding membrane areas. Data on the mechanical robustness of these varying membrane areas is also provided.

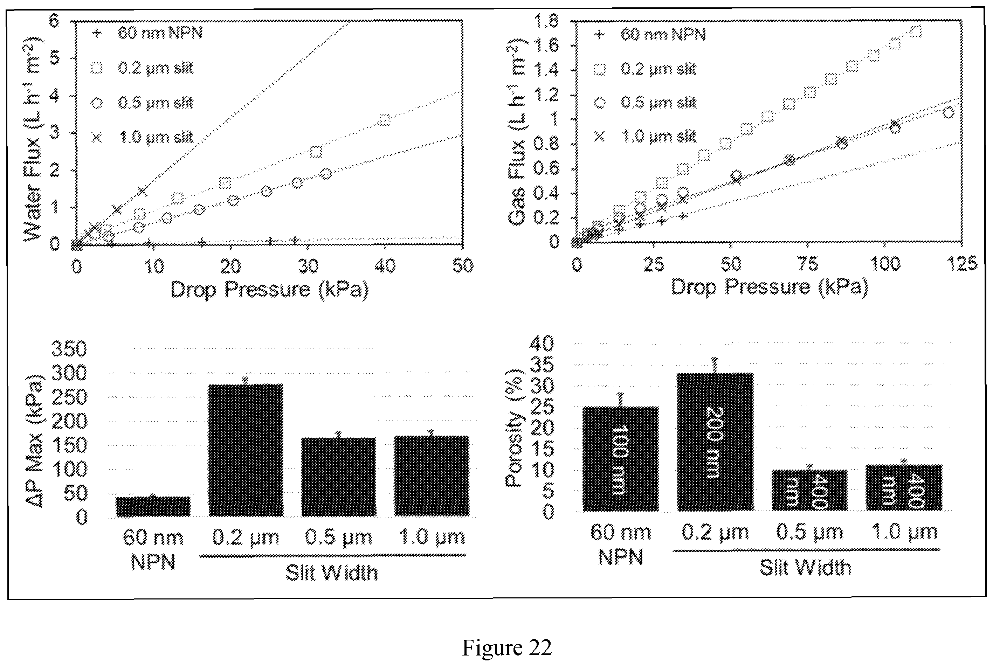

Figure 22 shows various membrane properties for microslit and nanoporous membrane filter formats, in terms of water and gas permeance, maximum differential pressure tolerance (a.k.a. "burst pressure"), and membrane physical properties (porosity, thickness, and average pore size)

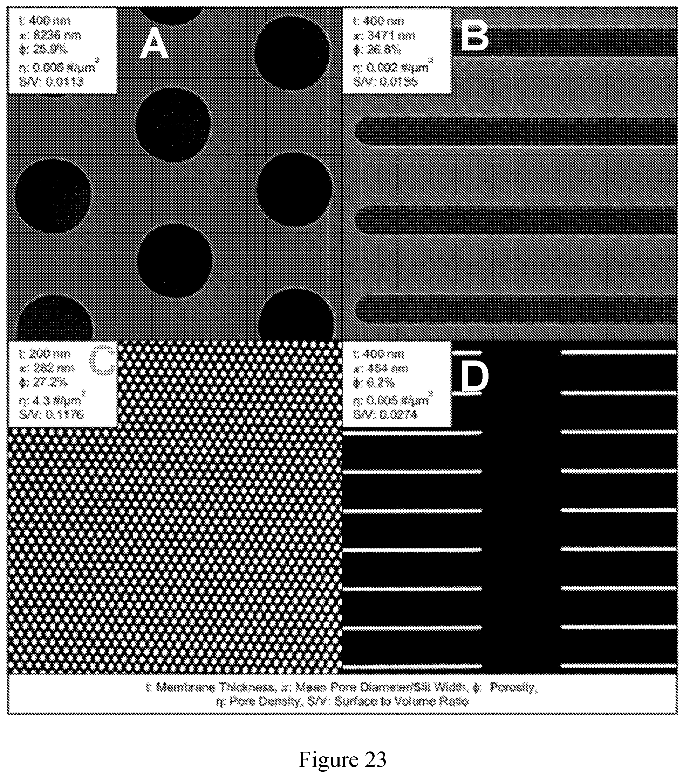

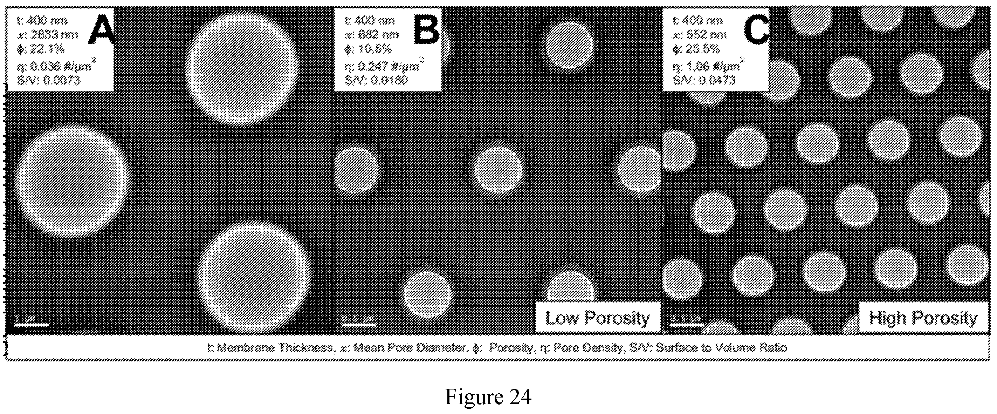

Figures 23 and 24 provide examples of microslit and microporous membrane formats, whereby permeability (by way of pore size and porosity) is provided.

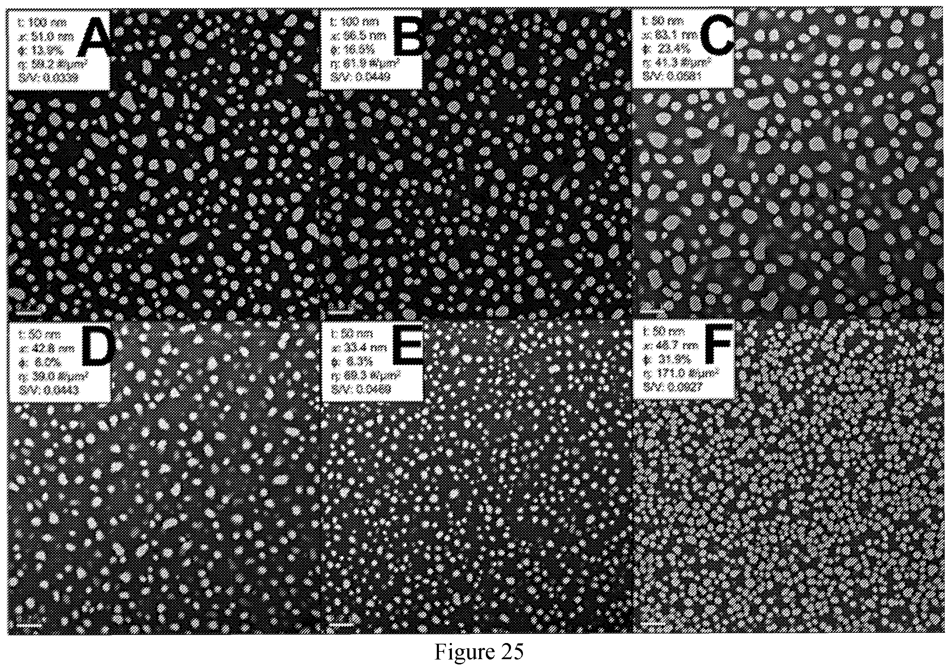

Figure 25 shows various nanoporous membrane filters with membranes of a various pore diameter and overall pore density

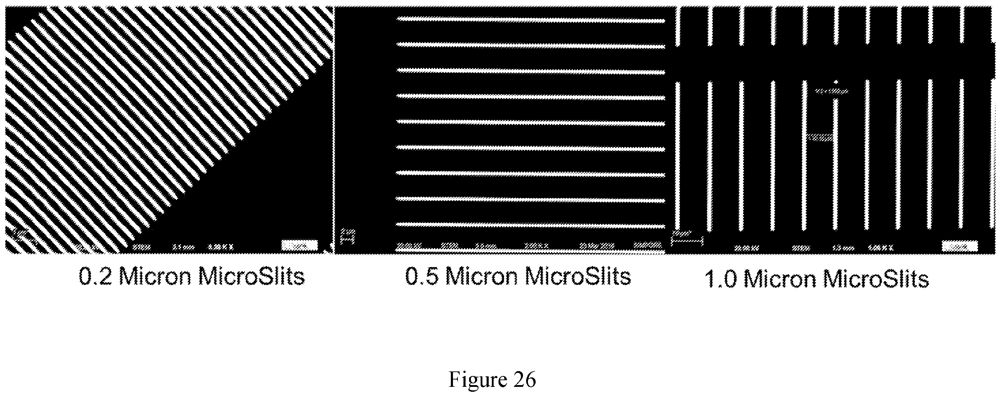

Figure 26 shows a comparison of 2 scanning electron micrographs for 0.2x10 µm, 0.5x50 µm, and 1x50 µm slit opening microslit filters.

### Non-Patent Citations

i. Leverett, L.B., Hellums, J.D., Alfrey, C.P., and Lynch, E.C. (1972). Red blood cell damage by shear stress. Biophys J 12, 257-273.
ii. Kawka, K., Madadkar, P., Umatheva, U., Shoaebargh, S., Medina, M.F.C., Lichty, B.D., Ghosh, R., and Latulippe, D.R. (2019). Purification of therapeutic adenoviruses using laterally-fed membrane chromatography. J Memb Sci 579, 351-358.

### Patent Citations

i. Huff, J. B. et al. Devices and methods for sample analysis. WO2016161402A1.
ii. Miller, B. and Rothberg, L. Method for biomolecular sensing and system thereof. U.S. Pat. No. 7,292,349.
iii. Shestopalov, A., McGrath, J. & Li, X. Methods for depositing a monolayer on a substrate. U.S. Pat. No. 9,089,819.
iv. Striemer, C.C., et al. Methods for Creating Fluidic Cavities by Transmembrane Etching Through Porous Membranes and Structures Made Thereby and Uses of Such Structures. U.S. Pat. No. 62/248,467.
v. Carter, J.A. and Roussie, J.A. Functionalized Silicon Nanomembranes and Uses Thereof. U.S. Pat. Appln. No. 62/614,232.
vi. Striemer, C.C. et al. Nanoporous Silicon Nitride Membranes, and Methods for Making and Using Such Membranes. U.S. Patent No. 9,789,239.
vii. Striemer, C.C. et al. "Free-Standing Silicon Oxide Membranes, and Methods of Making and Using Same. U.S. Patent No. 9,945,030.

The invention is defined by the appended claims.

## Claims

1. A method for enumerating and determining the composition of microplastic components of interest from a liquid sample, the method comprising:
contacting a liquid sample comprising a plurality of components with a microslit filter, such that one or more microplastic components of interest are retained by the microslit filter and undesired components permeate through the microslit filter;
optionally, washing the retained microplastic components of interest; and
measuring the quantity of retained microplastic components of interest to determine the concentration of the retained components of interest in the liquid sample; and
performing one or more first analytical assay on the one or more retained microplastic components of interest disposed on the microslit filter, wherein the components of interest are retained by the microslit filter during the first analytical assay, wherein the first analytical assay comprises determining the composition of the retained components of interest using infrared spectroscopy and/or Raman spectroscopy;
wherein the microslit filter comprises a silicon nitride membrane; and
wherein in the case of infrared spectroscopy the microslit filter comprises an infrared reflective layer comprising metal selected from Au, Pt, Ag, Ir, Rh and combinations thereof or in the case of Raman spectroscopy the microslit filter comprises a Raman-silent layer comprising MgF₂ or CaF₂.

2. The method of claim 1, wherein measuring the quantity of retained components of interest comprises optical imaging and/or optical diffraction.

3. The method of claim 1 or 2 further comprising preparing one or more upstream sample.

4. The method of any one of claims 1 to 3, further comprising performing a microplastic particle test on a liquid sample.

5. The method of any one of claims 1 to 4 further comprising:
successively contacting a liquid sample comprising a plurality of components with two or more microslit filters, such that two or more populations of components of interest are successively retained by the two or more microslit filters and undesired components permeate through the two or more microslit filters;
optionally, washing the retained components; and
measuring the quantity of the two or more populations of retained components to determine the concentration of the two or more populations of retained components in the liquid sample; performing one or more first analytical assay on the two or more populations of retained components of interest; and
wherein, optionally, contacting a liquid sample with two or more microslit filters comprises fluidic contact of the liquid sample with two or more first filtration membranes, two or more fluidic cavities, and two or more second filtration membranes.

6. The method of claim 5, wherein measuring the quantity of the two or more populations of retained components is performed as a single instance or as replicate instances for each component population.

7. The method of any one of claims 5 to 6, wherein contacting a liquid sample comprising a plurality of components with two or more microslit filters, such that two or more populations of components of interest are retained by the two or more microslit filters and undesired components permeate through the two or more microslit filters includes gravity flow, hydrostatic pressure, pumping, vacuum, centrifugation, gas pressurization, or tangential flow.

8. The method of any one of claims 1 to 7, wherein the coating has a thickness from 10 nm to 1,000 nm.

9. The method of any one of claims 1 to 8, wherein the infrared spectroscopy is Fourier-transformed infrared spectroscopy and the microslit filter comprises a coating comprising metal.

10. The method of any one of claims 1 to 9, wherein the Raman spectroscopy is surface-enhanced Raman spectroscopy.

11. The method of claim 1, wherein the liquid sample comprises a food, environmental, or industrial sample.

12. A device comprising at least one microslit filter, wherein each microslit filter comprises a plurality of openings configured such that one or more microplastic components of interest are retained by the microslit filter and undesired components permeate through the microslit filter,
wherein the device further comprises a light source and a detector configured to record optical signals of an optical modality,
wherein the light source and the detector are configured to record optical signals of an optical modality by performing Fourier-transformed infrared spectroscopy or Raman spectroscopy on microplastic components disposed on the microslit filter;
wherein the microslit filter comprises a silicon nitride membrane and wherein in the case of infrared spectroscopy the microslit filter comprises an infrared reflective layer comprising metal selected from Au, Pt, Ag, Ir, Rh and combinations thereof or in the case of Raman spectroscopy the microslit filter comprises a Raman-silent layer comprising MgF₂ or CaF₂.

## Patentansprüche

1. Verfahren zum Zählen und Bestimmen der Zusammensetzung von Mikroplastikkomponenten von Interesse aus einer Flüssigkeitsprobe, wobei das Verfahren Folgendes umfasst:
derartiges Inkontaktbringen einer Flüssigkeitsprobe, umfassend eine Vielzahl von Komponenten, mit einem Mikrospaltfilter, dass eine oder mehrere Mikroplastikkomponenten von Interesse von dem Mikrospaltfilter zurückgehalten werden und unerwünschte Komponenten durch den Mikrospaltfilter permeieren;
optional Waschen der zurückgehaltenen Mikroplastikkomponenten von Interesse; und
Messen der Menge der zurückgehaltenen Mikroplastikkomponenten von Interesse, um die Konzentration der zurückgehaltenen Komponenten von Interesse in der Flüssigkeitsprobe zu bestimmen; und
Durchführen einer oder mehrerer erster analytischer Untersuchungen an der einen oder den mehreren zurückgehaltenen Mikroplastikkomponenten von Interesse, die auf dem Mikrospaltfilter angeordnet sind, wobei die Komponenten von Interesse während der ersten analytischen Untersuchung von dem Mikrospaltfilter zurückgehalten werden, wobei die erste analytische Untersuchung ein Bestimmen der Zusammensetzung der zurückgehaltenen Komponenten von Interesse unter Verwendung von Infrarotspektroskopie und/oder Raman-Spektroskopie umfasst;
wobei der Mikrospaltfilter eine Siliziumnitridmembran umfasst; und
wobei in dem Fall der Infrarotspektroskopie der Mikrospaltfilter eine infrarotreflektierende Schicht umfasst, umfassend ein Metall, ausgewählt aus Au, Pt, Ag, Ir, Rh und Kombinationen davon, oder in dem Fall der Raman-Spektroskopie der Mikrospaltfilter eine Raman-silente Schicht umfasst, umfassend MgF₂ oder CaF₂.

2. Verfahren nach Anspruch 1, wobei das Messen der Menge der zurückgehaltenen Komponenten von Interesse ein optisches Abbilden und/oder eine optische Beugung umfasst.

3. Verfahren nach Anspruch 1 oder 2, ferner umfassend ein Herstellen einer oder mehrerer stromaufwärts gelegener Proben.

4. Verfahren nach einem der Ansprüche 1 bis 3, ferner umfassend ein Durchführen eines Mikroplastikpartikeltests an einer Flüssigkeitsprobe.

5. Verfahren nach einem der Ansprüche 1 bis 4, ferner umfassend:
derartiges aufeinanderfolgendes Inkontaktbringen einer Flüssigkeitsprobe, umfassend eine Vielzahl von Komponenten, mit zwei oder mehr Mikrospaltfiltern, dass zwei oder mehr Populationen von Komponenten von Interesse aufeinanderfolgend von den zwei oder mehr Mikrospaltfiltern zurückgehalten werden und unerwünschte Komponenten durch die zwei oder mehr Mikrospaltfilter permeieren;
optional Waschen der zurückgehaltenen Komponenten; und
Messen der Menge der zwei oder mehr Populationen der zurückgehaltenen Komponenten, um die Konzentration der zwei oder mehr Populationen der zurückgehaltenen Komponenten in der Flüssigkeitsprobe zu bestimmen;
Durchführen einer oder mehrerer erster analytischer Untersuchungen an den zwei oder mehr Populationen der zurückgehaltenen Komponenten von Interesse; und
wobei das Inkontaktbringen einer Flüssigkeitsprobe mit zwei oder mehr Mikrospaltfiltern optional einen fluidischen Kontakt der Flüssigkeitsprobe mit zwei oder mehr ersten Filtrationsmembranen, zwei oder mehr fluidischen Hohlräumen und zwei oder mehr zweiten Filtrationsmembranen umfasst.

6. Verfahren nach Anspruch 5, wobei das Messen der Menge der zwei oder mehr Populationen der zurückgehaltenen Komponenten als eine Einzelinstanz oder als Wiederholungsinstanzen für jede Komponentenpopulation durchgeführt wird.

7. Verfahren nach einem der Ansprüche 5 bis 6, wobei das derartige Inkontaktbringen einer Flüssigkeitsprobe, umfassend eine Vielzahl von Komponenten, mit zwei oder mehr Mikrospaltfiltern, dass zwei oder mehr Populationen von Komponenten von Interesse von den zwei oder mehr Mikrospaltfiltern zurückgehalten werden und unerwünschte Komponenten durch die zwei oder mehr Mikrospaltfilter permeieren, einen Schwerkraftfluss, hydrostatischen Druck, Pumpen, Vakuum, Zentrifugation, Gasdruckbeaufschlagung oder Tangentialfluss beinhaltet.

8. Verfahren nach einem der Ansprüche 1 bis 7, wobei die Beschichtung eine Dicke von 10 nm bis 1.000 nm aufweist.

9. Verfahren nach einem der Ansprüche 1 bis 8, wobei die Infrarotspektroskopie eine Fourier-transformierte Infrarotspektroskopie ist und der Mikrospaltfilter eine Beschichtung, umfassend Metall, umfasst.

10. Verfahren nach einem der Ansprüche 1 bis 9, wobei die Raman-Spektroskopie eine oberflächenverstärkte Raman-Spektroskopie ist.

11. Verfahren nach Anspruch 1, wobei die Flüssigkeitsprobe eine Lebensmittel-, Umwelt- oder Industrieprobe umfasst.

12. Vorrichtung, umfassend mindestens einen Mikrospaltfilter, wobei jeder Mikrospaltfilter eine Vielzahl von Öffnungen umfasst, die derart konfiguriert sind, dass eine oder mehrere Mikroplastikkomponenten von Interesse von dem Mikrospaltfilter zurückgehalten werden und unerwünschte Komponenten durch den Mikrospaltfilter permeieren,
wobei die Vorrichtung ferner eine Lichtquelle und einen Detektor umfasst, die dazu konfiguriert sind, optische Signale einer optischen Modalität aufzuzeichnen,
wobei die Lichtquelle und der Detektor dazu konfiguriert sind, durch Durchführen einer Fourier-transformierten Infrarotspektroskopie oder einer Raman-Spektroskopie an Mikroplastikkomponenten, die auf dem Mikrospaltfilter angeordnet sind, optische Signale einer optischen Modalität aufzuzeichnen;
wobei der Mikrospaltfilter eine Siliziumnitridmembran umfasst und wobei in dem Fall der Infrarotspektroskopie der Mikrospaltfilter eine infrarotreflektierende Schicht umfasst, umfassend ein Metall, ausgewählt aus Au, Pt, Ag, Ir, Rh und Kombinationen davon, oder in dem Fall der Raman-Spektroskopie der Mikrospaltfilter eine Raman-silente Schicht umfasst, umfassend MgF₂ oder CaF₂.

## Revendications

1. Procédé de dénombrement et de détermination de la composition de composants microplastiques d'intérêt à partir d'un échantillon liquide, le procédé comprenant :
la mise en contact d'un échantillon liquide comprenant une pluralité de composants avec un filtre à microfentes, de sorte qu'un ou plusieurs composants microplastiques d'intérêt soient retenus par le filtre à microfentes et que des composants indésirables traversent le filtre à microfentes ;
éventuellement, le lavage des composants microplastiques d'intérêt retenus ; et
la mesure de la quantité de composants microplastiques d'intérêt retenus pour déterminer la concentration des composants d'intérêt retenus dans l'échantillon liquide ; et
la réalisation d'un ou plusieurs premiers essais analytiques sur les un ou plusieurs composants microplastiques d'intérêt retenus disposés sur le filtre à microfentes, dans lequel les composants d'intérêt sont retenus par le filtre à microfentes pendant le premier essai analytique, dans lequel le premier essai analytique comprend la détermination de la composition des composants d'intérêt retenus à l'aide de spectroscopie infrarouge et/ou de spectroscopie Raman ;
dans lequel le filtre à microfentes comprend une membrane en nitrure de silicium ; et
dans lequel, dans le cas de la spectroscopie infrarouge, le filtre à microfentes comprend une couche réfléchissante infrarouge comprenant un métal choisi parmi Au, Pt, Ag, Ir, Rh et des combinaisons de ceux-ci, ou dans le cas de la spectroscopie Raman, le filtre à microfentes comprend une couche Raman-silencieuse comprenant MgF₂ ou CaF₂.

2. Procédé selon la revendication 1, dans lequel la mesure de la quantité de composants d'intérêt retenus comprend l'imagerie optique et/ou la diffraction optique.

3. Procédé selon la revendication 1 ou 2, comprenant également la préparation d'un ou plusieurs échantillons en amont.

4. Procédé selon l'une quelconque des revendications 1 à 3, comprenant également la réalisation d'un test de particules microplastiques sur un échantillon liquide.

5. Procédé selon l'une quelconque des revendications 1 à 4, comprenant également :
la mise en contact successive d'un échantillon liquide comprenant une pluralité de composants avec deux filtres à microfentes ou plus, de sorte que deux populations de composants d'intérêt ou plus soient successivement retenues par les deux filtres à microfentes ou plus et que des composants indésirables traversent les deux filtres à microfentes ou plus ;
éventuellement, le lavage des composants retenus ; et
la mesure de la quantité des deux populations de composants retenus ou plus pour déterminer la concentration des deux populations de composants retenus ou plus dans l'échantillon liquide ;
la réalisation d'un ou plusieurs premiers essais analytiques sur les deux populations de composants d'intérêt retenus ou plus ; et
dans lequel, éventuellement, la mise en contact d'un échantillon liquide avec deux filtres à microfentes ou plus comprend un contact fluidique de l'échantillon liquide avec deux premières membranes de filtration ou plus, deux cavités fluidiques ou plus et deux secondes membranes de filtration ou plus.

6. Procédé selon la revendication 5, dans lequel la mesure de la quantité des deux populations de composants retenus ou plus est réalisée en une seule fois ou en plusieurs fois pour chaque population de composants.

7. Procédé selon l'une quelconque des revendications 5 et 6, dans lequel la mise en contact d'un échantillon liquide comprenant une pluralité de composants avec deux filtres à microfentes ou plus, de sorte que deux populations de composants d'intérêt ou plus soient retenues par les deux filtres à microfentes ou plus et que des composants indésirables traversent les deux filtres à microfentes ou plus, comporte un écoulement par gravité, une pression hydrostatique, un pompage, un vide, une centrifugation, une pressurisation de gaz ou un écoulement tangentiel.

8. Procédé selon l'une quelconque des revendications 1 à 7, dans lequel le revêtement a une épaisseur de 10 nm à 1 000 nm.

9. Procédé selon l'une quelconque des revendications 1 à 8, dans lequel la spectroscopie infrarouge est une spectroscopie infrarouge à transformée de Fourier et le filtre à microfentes comprend un revêtement comprenant un métal.

10. Procédé selon l'une quelconque des revendications 1 à 9, dans lequel la spectroscopie Raman est une spectroscopie Raman exaltée de surface.

11. Procédé selon la revendication 1, dans lequel l'échantillon liquide comprend un échantillon alimentaire, environnemental ou industriel.

12. Dispositif comprenant au moins un filtre à microfentes, dans lequel chaque filtre à microfentes comprend une pluralité d'ouvertures configurées de sorte qu'un ou plusieurs composants microplastiques d'intérêt soient retenus par le filtre à microfentes et que des composants indésirables traversent le filtre à microfentes,
dans lequel le dispositif comprend également une source de lumière et un détecteur configurés pour enregistrer des signaux optiques d'une modalité optique,
dans lequel la source de lumière et le détecteur sont configurés pour enregistrer des signaux optiques d'une modalité optique en effectuant une spectroscopie infrarouge à transformée de Fourier ou une spectroscopie Raman sur des composants microplastiques disposés sur le filtre à microfentes ;
dans lequel le filtre à microfentes comprend une membrane en nitrure de silicium et dans lequel, dans le cas de la spectroscopie infrarouge, le filtre à microfentes comprend une couche réfléchissante infrarouge comprenant un métal choisi parmi Au, Pt, Ag, Ir, Rh et des combinaisons de ceux-ci, ou dans le cas de la spectroscopie Raman, le filtre à microfentes comprend une couche Raman-silencieuse comprenant MgF₂ ou CaF₂.
